# EUROPEAN PATENT SPECIFICATION

(11) **EP 4 359 754 B1**
(45) Date of publication and mention of the grant of the patent: **11.03.2026**
(21) Application number: 22735399.2
(22) Date of filing: 21.06.2022
(51) Int. Cl.: G01N 1/28, G01N 1/40

(54) **METHOD FOR DETECTING AT LEAST ONE ANALYTE IN A SAMPLE**
VERFAHREN ZUM NACHWEIS MINDESTENS EINES ANALYTEN IN EINER PROBE
PROCÉDÉ POUR DÉTECTER AU MOINS UN ANALYTE DANS UN ÉCHANTILLON

(30) Priority: 22.06.2021 EP 21180859
(43) Date of publication of application: 01.05.2024
(73) Proprietor: F. Hoffmann-La Roche AG, 4070 Basel (CH); Roche Diagnostics GmbH, 68305 Mannheim (DE)
(72) Inventor: REMPT, Martin, 82377 Penzberg (DE); SEITZ, Manuel Josef, 82377 Penzberg (DE); ZUTH, Christoph, 82377 Penzberg (DE)
(74) Representative: Epping, Claudia Viktoria
(86) International application number: PCT/EP2022/066865
(87) International publication number: WO 2022/268803

(56) References cited:
- WO-A1-2010/127059
- WO-A1-2012/170301
- US-A1- 2013 330 714
- BENJAMIN S. FREY ET AL: "Emerging trends in paper spray mass spectrometry: Microsampling, storage, direct analysis, and applications", MASS SPECTROMETRY REVIEWS., 6 September 2019 (2019-09-06), US, XP055674601, ISSN: 0277-7037, DOI: 10.1002/mas.21601

## Description

### Technical Field

The invention relates to a method for detecting at least one analyte in a sample, a sample preparation system with a kit. The devices and methods may be applied for measuring a mass of an analyte or a mass of fragments of the analyte for the purpose of identifying the analyte. Specifically, the devices and methods may be applied for the analysis of biological molecules such as proteins, peptides, oligonucleotides and small molecular compounds. Other applications, however, are also feasible.

### Background art

Paper spray ionization techniques typically utilize a paper substrate cut to a sharp point for which a sample of interest can be applied. Next, a spray solvent may be applied to the paper substrate that promotes both analyte extraction and ionization. Finally, a high voltage may be applied to the paper resulting in an electrospray-like ionization event at the paper tip. Although paper spray is relatively new in terms of ambient techniques, it has already been well-established and developed into commercial products.

Paper spray ionization techniques have been utilized in diverse clinical applications since its inception, which a large associated body of literature can corroborate. The majority of these applications have revolved around the analysis of biological matrices such as blood, plasma, and urine, but have also been extended to other complex material such as tissue biopsies, cell culture medium, tissue homogenate, and bacterial colonies. Paper spray ionization techniques commonly offer several advantages unique to clinical applications. The ability to use the collection medium, e.g. dried blood spot (DBS) paper, as the ionization medium greatly simplifies transport and storage of samples. Considering the single-use, disposable nature of these substrates, they also represent a low-cost consumable for clinicians.

In Manicke et al., Anal. Chem. 2015, 87, 6212- 6219, DOI: 10.1021/ acs.analchem.5b00884, a development of a paper spray mass spectrometry cartridge is described with integrated solid phase extraction for bioanalysis. A novel paper spray cartridge with an integrated solid phase extraction (SPE) column is described. The cartridge performs extraction and pre-concentration, as well as sample ionization by paper spray, from complex samples such as plasma. The cartridge allows for selective enrichment of target molecules from larger sample volumes and removal of the matrix, which significantly improved the signal intensity of target compounds in plasma samples by paper spray ionization.

Paper tip formed structures may be used as spray cones. In US 2017 /0053788 Al a paper cone tip, having a triangular-pyramidal shape with a vertex angle of 12.5° to 45°, and a paper cone spray ionization mass spectrometry (PCSI MS) method using a paper cone tip is described. The paper cone spray ionization mass spectrometry (PCSI MS) method includes: preparing the paper cone tip having a triangular-pyramidal shape; placing a measuring sample in the paper cone tip and locating the paper cone tip in front of a mass spectrometer; and adding a spraying solvent to the paper cone tip and applying a voltage thereto.

In WO 2013/102670 Al an electrostatic spray ionization method for spraying a liquid layer from an insulating plate is described. The plate is arranged between two electrodes. A constant high voltage power supply is provided and an electric circuit is used to charge and discharge locally a surface of the liquid layer on the insulating plate by applying the power supply between the electrodes.

In WO 00/30167 A1 a MEMS device with an overhanging "polymer" capillary is described which provides vital and significant improvements in interfacing a MEMS electrospray nozzle to an MS inlet or other macroscopic instrumentation. The fabrication methodology associated therewith is easily expanded to include built-in micro particle filters and centimeter long serpentine micro channels provided on-chip and fabricated using a low temperature process.

Further, wetted porous material and polymer based material have so far been used in combination with dispensing units.

In WO 2010/127059 Al systems and methods for mass spectrometry analysis of samples are described. In certain embodiments, a mass spectrometry probe including at least one porous material connected to a high voltage source is provided, in which the porous material is discrete from a flow of solvent.

Further, a paper was used as a sampling device for microorganisms followed by paper based electrospray ionization mass spectrometry. Specifically, in US 2013/0330714 Al systems and methods for mass spectrometry analysis of microorganisms in samples are described.

Moreover, a pre-coated filter paper was used as solid phase extraction material. This method may also be referred to as coated blade spray technique.

In Pawliszyn et al., Angew. Chem. Int. Ed. 2014, 53, 14503 -14507, DOI: 10.1002/anie.201407057, a development of coated blade spray ionization mass spectrometry for the quantitation of target analytes present in complex matrices is described. Coated blade spray (CBS) is a technology based on solid-phase microextraction (SPME) that has been designed for the quick extraction/cleanup of analytes from complex matrices and direct desorption/ionization under ambient mass spectrometry conditions. The entire analytical process can be completed in less than 3 min and enables limits of quantitation in the low picogram-per-milliliter region to be reached.

In Ouyang et al., Analytical Chemistry, Vol. 82, No. 6, 2463-2471, DOI: 10.1021/ac902854g, a development, characterization, and application of paper spray ionization is described. Paper spray is developed as a direct sampling ionization method for mass spectrometric analysis of complex mixtures. Ions of analyte are generated by applying a high voltage to a paper triangle wetted with a small volume (<10 µL) of solution. Samples can be preloaded onto the paper, added with the wetting solution, or transferred from surfaces using the paper as a wipe.

In Badu-Tawiah et al., Mass Spectrometry Reviews, 2020, 39, 336-370, DOI 10.1002/mas.21601, emerging trends in paper spray mass spectrometry including mi-crosampling, storage, direct analysis and applications are described.

A magnetic bead based clean up procedure for complex matrices is described in US 2009/0090855 A1. Specifically, in US 2009/0090855 Al, a novel procedure of preparing samples for analysis by way of mass spectrometry, preferably LC-MS/MS is described. Accordingly, functionalized magnetic particles with a hydrophobic surface were used for extracting low molecular weight compounds from complex liquid biological samples such as plasma, serum, whole blood or hemolyzed blood. The method of the invention includes (a) contacting the sample with an amount of functionalized magnetic particles with a hydrophobic surface, (b) incubating the sample and the particles, thereby adsorbing the compound to the hydrophobic surface, (c) separating the particles by applying a magnetic field and removing the liquid, (d) optionally washing the particles, (e) eluting the compound from the particles.

WO 2012/170301 A1 describes systems, and methods for ion generation using wetted porous materials. Specifically, WO 2012/170301 A1 describes a sampling cassette. The cassette comprises: a hollow housing comprising an inside configured to hold a solid porous substrate, at least one inlet, an outlet, and an electrode, wherein the housing is configured such that the inlet is in fluid communication with the substrate held inside the housing and the electrode is in contact with the substrate held inside the housing.

WO 2012/170301 A1 relates to cassettes, systems, and methods for ion generation using wetted porous materials.

Despite the advantages achieved by the above-mentioned devices, several technical challenges remain.

Biological samples such as serum, plasma and urine as well as the respective analytes commonly mostly exhibit very low concentration ranges to be biologically active. Exemplarily, estradiol exhibits a biologically active concentration range down to 0.5 pg/ml. In addition, biological samples often comprise a variety of analysis disturbing matrix components. To get such biological substances prepared for conducting an analysis method an effective clean up procedure needs to be established. For mass spectrometry, an automated way comprising microparticle handling and using different washing/elution steps is desired. The elution of an analyte from a microparticle which is used as clean up agent may result in a dilution of the analyte. Further, mostly due to pipetting reasons, the whole elution volume cannot be applied to the analytical system. Further, paper-based ionization for mass spectrometry gives an alternative way to use paper strips instead of electrospray ionization capillary as voltage/solvent interface in front of a mass spectrometer entrance and ion generation unit. As shortcoming with paper to being used within the paper spray ionization technique, a poor sample clean up ability and volume applying technology can be seen.

### Problem to be solved

It is therefore desirable to provide a method for detecting at least one analyte in a sample, a sample preparation system with a kit which at least partially address the above-mentioned technical challenges. Specifically, an effective clean up procedure of a sample shall be provided.

### Summary

This problem is addressed by a method for detecting at least one analyte in a sample and a sample preparation system with a kit with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restriction regarding the scope of the invention and without any restriction regarding the possibility of combining the features introduced in such way with other optional or non-optional features of the invention.

In a first aspect of the present invention, a method for detecting at least one analyte in a sample is disclosed.

The method comprises the following steps which specifically may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. Specifically, steps d) and e) may be performed simultaneously or in a timely overlapping fashion. The method may comprise further method steps which are not listed.

The method comprises the following steps:
a) providing at least one sample having at least one analyte;
b) incubating the sample with microparticles having at least one surface whereby the analyte is adsorbed on the surface of the microparticles and an analyte-microparticle-complex is formed;
c) providing at least one fiber sheet material having at least one tip and contacting the tip of the fiber sheet material to the sample comprising the analyte-microparticle-complex, whereby at least the analyte-microparticle-complex is sucked into the tip of the fiber sheet material;
d) contacting the tip of the fiber sheet material to a port of an analytical device;
e) adding an extracting solvent to the fiber sheet material and applying an electrical voltage to the fiber sheet material whereby ions of the analyte are generated and are expelled from the tip of the fiber sheet material; and
f) detecting the at least one analyte with the analytical device.

The term "analyte" as used herein is a broad term and is to be given its ordinary and customary meaning to a person of ordinary skill in the art and is not to be limited to a special or customized meaning. The term specifically may refer, without limitation, to an arbitrary chemical or biological substance or species, such as a molecule or a chemical compound, to be detected and/or measured. Specifically, a presence, an absence, a concentration and/or an amount of the analyte in a sample may be detected or measured. Specifically, the analyte may be a biological molecule or macromolecule. The analyte may be selected from the group consisting of: a steroid, specifically a ketosteroid, specifically a secosteroid; a therapeutically active substance, specifically an antibiotic, specifically a local anesthetic, specifically Lidocaine; a peptide, specifically Cyclosporine A; a protein; a metabolite, specifically a nucleotide, specifically Adenosine 5'-monophosphate; a nucleic acid; an amino acid; a hormone; a fatty acid; a lipid; a carbohydrate. Further, the analyte may be a molecule characteristic of a certain modification of another molecule or a substance that has been internalized by an organism or a metabolite of such a substance or a combination thereof. However, also different kinds of analytes may be feasible. Further substances, in particular molecules with different functional groups and/or different properties, e.g. hydrophobic and hydrophilic molecules may serve as analytes. A concentration of the analyte in the sample may be in the range of 0.01 pg/ml to 1 mg/ml, preferably of 0.05 pg/ml to 50 µg/ml, most preferably of 0.1 pg/ml to 10 µg/ml. However, also other concentrations may be feasible. The analyte may have a molar mass from 10 Da to 10000 Da, preferably from 50 Da to 3000 Da, most preferably from 100 Da to 2000 Da.

The term "detecting an analyte" as used herein is a broad term and is to be given its ordinary and customary meaning to a person of ordinary skill in the art and is not to be limited to a special or customized meaning. The term specifically may refer, without limitation, to a quantitative and/or qualitative determination of at least one analyte in an arbitrary sample. The quantitative and/or qualitative determination of the analyte in the sample may be a result or an intermediate result of a detection process that may comprise at least one measurement step as well as further steps such as at least one preparation step and/or at least one analyzing step. As part of the detection process at least one measurement value may be generated, specifically a measurement value regarding the presence, absence, concentration or amount of the analyte in the sample.

The term "sample" as used herein is a broad term and is to be given its ordinary and customary meaning to a person of ordinary skill in the art and is not to be limited to a special or customized meaning. The term specifically may refer, without limitation, to an arbitrary sample such as a biological sample, also called test sample, a quality control sample, an internal standard sample. The sample may comprise one or more analytes of interest. The sample may specifically be a liquid sample, in particular a liquid sample comprising at least one biological material. For example, the sample may be selected from the group consisting of: a physiological fluid, including blood, serum, plasma, saliva, ocular lens fluid, cerebral spinal fluid, sweat, urine, milk, ascites fluid, mucous, synovial fluid, peritoneal fluid, amniotic fluid, tissue, cells or the like. The sample may be used directly as obtained from the respective source or may be subject of a pretreatment and/or sample preparation workflow. Further, the sample may undergo one or more treatment steps as part of the method for detecting the analyte in the sample. Thus, at least one property of the sample, e.g. the sample composition, the sample volume, the analyte concentration or other sample properties, may change during the method for detecting the analyte. Specifically, one or more components, such as chemical or biological compound of the sample, may be removed from the sample as part of one or several preparation steps. Specific sample treatment steps, that may in particular be part of step b) of the method, will be described in more detail further below. Further, the initially liquid sample may undergo a drying process, particularly after having been sucked into the tip of the fiber sheet material as part of step c). As part of the drying process some or all of the liquid of the sample, e.g. some or all of a solvent, may evaporate as described in further detail below.

As outlined above, in step a) the at least one sample having the at least one analyte is provided. The term "providing", as used herein, is a broad term and is to be given its ordinary and customary meaning to a person of ordinary skill in the art and is not to be limited to a special or customized meaning. The term specifically may refer, without limitation, to a process of making available one or more needed objects.

As outlined above, in step b) of the method, the sample is incubated with the microparticles having the at least one surface whereby the analyte is adsorbed on the surface of the microparticles and the analyte-microparticle-complex is formed.

The term "microparticles" as used herein is a broad term and is to be given its ordinary and customary meaning to a person of ordinary skill in the art and is not to be limited to a special or customized meaning. The term specifically may refer, without limitation, to an arbitrary particulate matter of microscopic size. The microparticles may have a mean diameter in the range from 100 nm to 100 µm, specifically from 200 nm to 50 µm. The microparticles may also be referred to as beads. The microparticles may be of spherical or globular shape. However, slight derivations from the spherical or globular shape may be feasible. As outlined above, the microparticles have the at least one surface. The term "surface" as used herein is a broad term and is to be given its ordinary and customary meaning to a person of ordinary skill in the art and is not to be limited to a special or customized meaning. The term specifically may refer, without limitation, to an entirety of areas which delimit an arbitrary body from the outside. Thus, the body may have a plurality of surfaces. Specifically, the microparticles may have a core surrounded by the surface. The surface and the core may comprise different materials. Further, the surface and the core may have different properties. Exemplarily, the core may be magnetic. The surface may be configured for capturing molecules, e.g. a broad range of polar to apolar molecules, when the microparticles are incubated with a sample comprising such molecules.

In particular, the microparticles may be selected from the group consisting of: magnetic microparticles, specifically magnetic microparticles having a magnetic core and a modified surface; silica microparticles, specifically silica microparticles having a silica core and a modified surface; melamine resin microparticles, specifically melamine resin microparticles having a melamine resin core and a modified surface; poly(styrene) based microparticles, specifically poly(styrene) based microparticles having a poly(styrene) core and a modified surface; poly(methyl methacrylate) microparticles, specifically poly(methyl methacrylate) microparticles having a poly(methyl methacrylate) core and a modified surface. However, also other particles may be feasible. The melamine resin microparticles may have a mean diameter of 500 nm to 20 µm, preferably of 2 µm to 4 µm, most preferably of 3 µm. The poly(styrene) based microparticles may have a mean diameter of 500 nm to 50 µm, preferably of 2 µm to 4 µm, most preferably of 3 µm. The poly(methyl methacrylate) microparticles may have a mean diameter of 500 nm to 50 µm, preferably of 2 µm to 4 µm, most preferably of 3 µm. The modified surface of the magnetic microparticles may be a modified poly(styrene) surface and the magnetic microparticles may have a mean diameter of 5 µm to 50 µm, preferably of 10 µm to 30 µm, most preferably of 20 µm. The modified surface of the magnetic microparticles may be a silica surface and the magnetic microparticles may have a mean diameter of 100 nm to 1000 nm, preferably of 200 nm to 500 nm, most preferably of 300 nm. The modified surface of the silica microparticles may be a cyanopropyl silane functionalized surface and the silica microparticles may have a mean diameter of 5 µm to 100 µm, preferably of 20 µm to 80 µm, most preferably of 40 µm. Also other dimensions may be feasible.

The term "incubation" as used herein is a broad term and is to be given its ordinary and customary meaning to a person of ordinary skill in the art and is not to be limited to a special or customized meaning. The term specifically may refer, without limitation, to a mixing of at least two substances and/or to an addition of at least one substance to another. Specifically, a solid or particulate matter may be added to and/or mixed with a sample of liquid. Apart from the process of adding and/or mixing, the incubation may further comprise a period of time referred to as incubation time. During the incubation time one of the two substances may be adsorbed on a surface of the other one of the two substances. During the incubation time further conditions, such as temperature and/or other conditions, may be chosen e.g. to favor the desired adsorption. Thus, in step b) the microparticles may be added to the sample and may optionally be mixed with the sample. In step b), the sample may be incubated with the microparticles with an incubation time of 1 s to 60 min, preferably of 1 min to 30 min, most preferably of 3 min to 12 min. However, also other durations may be feasible.

The term "being adsorbed on a surface" as used herein is a broad term and is to be given its ordinary and customary meaning to a person of ordinary skill in the art and is not to be limited to a special or customized meaning. The term specifically may refer, without limitation, to a result of a process during which atoms, ions or molecules forming part of a gas or liquid accumulate at a surface of an object of solid or particulate matter. The atoms, ions or molecules that may initially be distributed throughout the gas or liquid may be attracted by the surface of the solid matter or the particulate matter in the process of adsorption.

The term "analyte-microparticle-complex" as used herein is a broad term and is to be given its ordinary and customary meaning to a person of ordinary skill in the art and is not to be limited to a special or customized meaning. The term specifically may refer, without limitation, to an ensemble comprising at least one microparticle and at least one analyte, specifically one microparticle and a plurality of analytes. The microparticle and the analyte, specifically the analytes, forming the complex may be reversibly associated. Thus, the components of the complex may, at least under certain conditions, leave the complex or dissociate from the complex. The analyte-microparticle-complex may form on the basis of at least one force of attraction between the microparticle and the analyte. In particular, the force of attraction may act between the surface of the microparticles and the analyte. Thus, the analyte that may initially be distributed in the sample, specifically in a liquid phase of the sample, may accumulate in a process of adsorption at the surface of the microparticles. The forces of attraction may include van der Waals forces and electrostatic attraction. Other forces of attraction are feasible. Specifically, at least one chemical bond may be formed between the microparticle and the analyte, specifically between the surface of the microparticle and the analyte, as part of the formation of the analyte-microparticle-complex. The analyte-microparticle-complex may also be referred to as analyte loaded microparticles.

As outlined above, in step b), the sample is incubated with the microparticles having the at least one surface whereby the analyte is adsorbed on the surface of the microparticles and the analyte-microparticle-complex is formed. In this context, the expression can be understood that a plurality of analyte-microparticle-complexes are formed. Thus, in step b), the sample may be incubated with the microparticles having the at least one surface whereby the analyte is adsorbed on the surface of the microparticles and the analyte-microparticle-complexes are formed. Further, in step c), the at least one fiber sheet material having the at least one tip may be provided and the tip of the fiber sheet material may be contacted to the sample comprising the analyte-microparticle-complexes, whereby at least the analyte-microparticle-complexes are sucked into the tip of the fiber sheet material.

The method may further comprise the following steps:
b1) separating of the analyte-microparticle-complex, specifically of the analyte-microparticle-complexes, from further components of the sample; and
b2) removing the further components of the sample from the analyte-microparticle complex, specifically from the analyte-microparticle complexes.

The step b1) may exemplary comprise a centrifugation of the sample. Further, additionally or alternatively, the step b1) may comprise a magnetic based separation. Specifically, the microparticles may be magnetic microparticles and the step b1) may comprise the magnetic based separation. The step b2) may specifically include a removal of a supernatant by a liquid handling device such as by a pipette.

The magnetic based separation may exemplarily comprise a sucking up of at least the analyte-microparticle-complex by a liquid handling device, specifically by a pipette. Thereafter, a magnetic separator may be brought in close proximity to the liquid handling device. Exemplarily, the magnetic separator may touch an outer surface of the liquid handling device. The magnetic separator may be configured for holding the analyte-microparticle-complex within an interior space of the liquid handling device. Thus, residual components of the sample such as the matrix may be separated from the analyte-microparticle-complex and may be removed. Optionally, washing steps may be conducted. For this purpose, the analyte-microparticle-complex may be washed with at least one washing solvent followed by a further sucking up of at least the analyte-microparticle-complex by the liquid handling device and by holding the analyte-microparticle-complex within the interior space of the liquid handling device by the magnetic separator. Thereafter, the tip of the fiber sheet material may be contacted to the analyte-microparticle-complex whereby at least the analyte-microparticle-complex is sucked into the tip of the fiber sheet material. For this purpose, the tip of the fiber sheet material may be brought close to a tip of the liquid handling device. However, also other magnetic based separation techniques may be feasible.

Further, the method may comprise the following step:
b3) washing the analyte-microparticle-complex, specifically the analyte-microparticle-complexes.

Specifically, the analyte-microparticle-complex may be washed with a washing solvent. A composition of the washing solvent may be chosen such that the analyte remains bound to the microparticle. The washing solvent may be or may comprise deionized water. Further, the washing solvent may comprise a mixture of water, one or more buffering salts, one or more pH-adjusting additives and/or one or more organic solvents. The organic solvent may be selected from the group consisting of: methanol, ethanol, isopropanol, acetonitrile. A content of the organic solvent may be 0 vol% to 10 vol%. The step b3) may be repeated at least two times, preferably at least three times.

The term "fiber sheet material" as used herein is a broad term and is to be given its ordinary and customary meaning to a person of ordinary skill in the art and is not to be limited to a special or customized meaning. The term specifically may refer, without limitation, to an arbitrary material which is made of or comprises a plurality of fibers and may, additionally, have an essentially flat shape. The fibers may be natural fibers directly derived from living organisms or may be synthetic fibers manufactured through chemical synthesis. The fibers may have a length which exceeds its width at least by a factor of 5, at least a factor of 10, or even at least a factor of 20 or more. The fiber sheet material may be selected from the group consisting of: cellulose, specifically a cellulose chromatography paper, specifically a cellulose acetate membrane, a glass filter membrane. However, also other materials may be feasible. The fiber sheet material may specifically be stable toward organic solvents, specifically toward the extracting solvent. The fiber sheet material may have a grammage of 10 g/m² to 3000 g/m², preferably of 20 g/m² to 1000 g/m², most preferably of 80 g/m² to 700 g/m². However, also other grammages may be feasible. The fiber sheet material may have an elongated shape. Specifically, the fiber sheet material may be provided as a strip.

The term "tip" as used herein is a broad term and is to be given its ordinary and customary meaning to a person of ordinary skill in the art and is not to be limited to a special or customized meaning. The term specifically may refer, without limitation, to a sharp end of an arbitrary element, specifically of an elongated element. The tip of the fiber sheet material may specifically have a triangular shape. Further, the tip may have a tip angle of 5° to 90°, preferably of 20° to 80°. The tip angle may specifically refer to an opening angle of a triangle. However, also other shapes may be feasible.

As outlined above, the tip of the fiber sheet material is contacted to the sample comprising the analyte-microparticle-complex. The term "contacting", in this context, may specifically refer to an arbitrary process wherein one element is brought into direct contact with another element. Thus, the two elements may touch each other. Exemplarily, the tip of the fiber sheet material may simply be plunged into the sample comprising the analyte-microparticle-complex. However, in principal, also other techniques may be feasible.

As outlined above, in step c), at least the analyte-microparticle-complex is sucked into the tip of the fiber sheet material. Thus, optionally, also other components of the sample may be sucked into the tip of the fiber sheet material. The term "sucking" may specifically refer to a pulling of the sample comprising the analyte-microparticle-complex into the fiber sheet material, specifically through the tip of the fiber sheet material, specifically in a particular direction.

The method may, optionally, further comprise the following step:
c1) drying the fiber sheet material such that liquid of the sample evaporates.

The step c1) may comprise a natural drying of the fiber sheet material, e.g. a drying in the open air. Further, additionally or alternatively, the c1) may comprise a drying of the fiber sheet material under vacuum. However, it is not absolutely necessary to dry the fiber sheet material completely. Further, step f) may also be conducted in case the fiber sheet material comprises residuals of the washing solvent. Before step d) is conducted, the method may further comprise a storing of the fiber sheet material. Specifically, step c1) may be conducted before the fiber sheet material is stored.

As outlined above, in step d), the tip of the fiber sheet material is contacted to a port of an analytical device. The term "contacting", in this context, may specifically refer to an arbitrary process wherein one element is brought into contact with another element. The contacting may refer to a direct contacting or to an indirect contacting. Specifically, the tip of the fiber sheet material may be contacted to the port of an analytical device such that a material exchange, specifically a gas exchange, occurs. Thus, exemplarily, the tip of the fiber sheet material and the port of the analytical device may touch each other. Exemplarily, the tip of the fiber sheet material may simply be placed on a surface of the port. However, the tip of the fiber sheet material and the port of the analytical device may also be arranged in a distance to each other. Specifically, the tip of the fiber sheet material and the port of the analytical device may be arranged in a distance of 0.1 cm to 5 cm, preferably in a distance of 0.2 cm to 3 cm, most preferably in a distance of 0.5 cm to 2 cm. Further, the tip of the fiber sheet material and the port of the analytical device may be arranged in a distance of less than 2 cm, preferably in a distance of less than 1 cm, most preferably in a distance of less than 0.5 cm to each other.

Specifically, the tip of the fiber sheet material may be contacted to the port of the analytical device by one of the following procedures selected from the group consisting of: the tip of the fiber sheet material is directly contacted to the port of the analytical device, specifically the tip of the fiber sheet material and the port of the analytical device touch each other; the tip of the fiber sheet material is indirectly contacted to the port of the analytical device, specifically the tip of the fiber sheet material and the port of the analytical device are arranged in a distance to each other, preferably in a distance of 0.1 cm to 5 cm, preferably in a distance of 0.2 cm to 3 cm, most preferably in a distance of 0.5 cm to 2 cm.

The term "analytical device" as used herein is a broad term and is to be given its ordinary and customary meaning to a person of ordinary skill in the art and is not to be limited to a special or customized meaning. The term specifically may refer, without limitation, to a device configured for carrying out at least one analytical measurement with the sample or at least one component of the sample. As a result of the measurement or as part of the measurement the analytical device may generate at least one measurement result characteristic for the sample and/or the at least one component, specifically at least one measurement result regarding the analyte. **In** particular, analyte-related information regarding the analyzed sample may be derived or may be derivable from the analytical measurement. The information may e.g. regard the presence, the absence, the concentration or the amount of analyte present in the sample. In particular, the analytical device may be configured for detecting the analyte in the sample. Specifically, the analytical device may be selected from the group consisting of: a mass spectrometer; an ion mobility device; a combination of a mass spectrometer and an ion mobility device. However, also other devices may be feasible.

The term "mass spectrometer" as used herein is a broad term and is to be given its ordinary and customary meaning to a person of ordinary skill in the art and is not to be limited to a special or customized meaning. The term specifically may refer, without limitation, to an arbitrary analytical device which is configured for measuring a mass-to-charge ratio of ions. Measurement results may specifically be presented as a mass spectrum, e.g. a plot of intensity as a function of the mass-to-charge ratio.

The term "ion mobility device" as used herein is a broad term and is to be given its ordinary and customary meaning to a person of ordinary skill in the art and is not to be limited to a special or customized meaning. The term specifically may refer, without limitation, to an arbitrary analytical technique which is configured to separate and identify ionized molecules in a gas phase based on their mobility in a carrier buffer gas. The ion mobility device may specifically be coupled to the mass spectrometer, specifically in order to achieve a multi-dimensional separation. The ion mobility device may be configured for detecting at least one drift time of the ion through the ion-mobility spectrometry cell. The ion mobility device may also be referred to as one ion-mobility spectrometry device.

The term "port" as used herein is a broad term and is to be given its ordinary and customary meaning to a person of ordinary skill in the art and is not to be limited to a special or customized meaning. The term specifically may refer, without limitation, to an arbitrary component or subunit of the analytical device configured for receiving, accepting or making contact to the sample to be analyzed by the analytical device.

As outlined above, in step e), an extracting solvent is added to the fiber sheet material. In step e), the extracting solvent may be applied onto the fiber sheet material. Specifically, the extracting solvent may be pipetted onto the fiber sheet material. Further, additionally or alternatively, the extracting solvent may be sprayed onto the fiber sheet material. Specifically, in step e), the fiber sheet material may be in contact with a capillary and a continuous flow of the extracting solvent is provided to the fiber sheet material via the capillary. The continuous flow of the extracting solvent may have a flow rate of 1 µL/min to 50 µL/min, preferably of 10 µL/min to 30 µL/min, most preferably of 20 µL/min. Specifically, the extracting solvent may be applied on a region of the fiber sheet material which differs from the tip of the fiber sheet material. The extracting solvent may move by capillary forces towards the tip of the fiber sheet material. The paper sheet material may specifically comprise a front side and a rear side. The extracting solvent may be applied on the rear side of the paper sheet material.

The term "extracting solvent" as used herein is a broad term and is to be given its ordinary and customary meaning to a person of ordinary skill in the art and is not to be limited to a special or customized meaning. The term specifically may refer, without limitation, to an arbitrary solvent which is utilized for an arbitrary separation process which includes a separation of a substance from another substance. The extracting solvent may be an organic solvent or may comprise an organic solvent. The organic solvent may be selected from the group consisting of: acetonitrile, methanol, isopropanol, ethanol, tetrahydrofuran. Further, the extracting solvent may comprise one or more additives. Up to 10% dimethyl sulfoxide or 0.1% formic acid, acetic acid, trifluoroacetic acid, ammonium fluoride, ammonium hydroxide or triethylamine may be added as additives. Further, the extracting solvent may comprise deionized water. A water content of the extracting solvent may be 0% to 20%. Due to the high organic content of the extracting solvent, an extraction of the analytes from the microparticles and, thus, a separation of the analytes from the microparticles, may be ensured.

Further, as outlined above, in step e), an electrical voltage is applied to the fiber sheet material whereby ions of the analyte are generated and are expelled from the tip of the fiber sheet material. The ions of the analyte may be applicable for an analysis via a mass spectrometer. Specifically, in step e), an electrical voltage of 1 kV to 10 kV, preferably of 2 kV to 5 kV, most preferably of 3 kV, may be applied. Specifically, the electrical voltage is applied to the paper sheet material via contact with at least one clip, specifically at least one copper clip. The clip may be connected to a voltage source supply, specifically a high voltage source supply. The port of the analytical device may comprise an electrode and an electric field may be produced through the paper sheet material when the electrical voltage is applied. The extracting solvent may be configured for separating the analyte from the microparticle. Further, the analyte may be ionized and may be sprayed from the tip of the paper sheet material. The microparticle may remain on the paper sheet material.

A transport of the analyte-microparticle-complex may occur by the extracting solvent and by an electric field.

In a further aspect of the present invention, a sample preparation system, specifically a continuous sample preparation system, is disclosed. The sample preparation system is configured for conducting the method for detecting at least one analyte as outlined above or as will further be described below in more detail.

The term "sample preparation system" as used herein is a broad term and is to be given its ordinary and customary meaning to a person of ordinary skill in the art and is not to be limited to a special or customized meaning. The term specifically may refer, without limitation, to an arbitrary system which is configured to prepare a sample for using a sample for a special purpose such as for conducting an analytical measurement. During sample preparation, at least one property of the sample, e.g. the sample composition, the sample volume, the analyte concentration or other sample properties, may change. The term "continuous sample preparation system" as used herein is a broad term and is to be given its ordinary and customary meaning to a person of ordinary skill in the art and is not to be limited to a special or customized meaning. The term specifically may refer, without limitation, to an arbitrary sample preparation system which is configured for preparing a plurality of samples successively, preferably without cessation between the preparations.

The sample preparation system comprises:
- at least one vessel, wherein the vessel is configured for providing at least one sample having at least one analyte;
- at least one liquid handling system, wherein the liquid handling system is configured for adding microparticles to the vessel whereby the analyte is adsorbed on a surface of the microparticles and an analyte-microparticle-complex is formed;
- at least one holder, wherein the holder is configured for holding at least one fiber sheet material having at least one tip, wherein the holder is further configured for contacting the tip of the fiber sheet material to the sample comprising the analyte-microparticle-complex;

- at least one analytical device, specifically at least one mass spectrometer, wherein the analytical device is configured for detecting the at least one analyte, wherein the analytical device comprises at least one port;
- at least one sample changer, wherein the sample changer is configured for transferring the holder holding the fiber sheet material to the port of the analytical device, such that the tip of the fiber sheet material is in contact with the port;
- at least one electrical contact, wherein the electrical contact is configured for applying an electrical voltage to the fiber sheet material; and
- at least one extracting solvent supply, wherein the extracting solvent supply is configured for wetting the fiber sheet material with at least one extracting solvent.

The term "vessel" as used herein is a broad term and is to be given its ordinary and customary meaning to a person of ordinary skill in the art and is not to be limited to a special or customized meaning. The term specifically may refer, without limitation, to an arbitrary device which is configured for receiving and holding at least one substance, specifically at least one liquid substance. The vessel may have an arbitrary shape.

The term "liquid handling system" as used herein is a broad term and is to be given its ordinary and customary meaning to a person of ordinary skill in the art and is not to be limited to a special or customized meaning. The term specifically may refer, without limitation, to an arbitrary device which is configured for applying liquid, specifically a defined or desired amount of liquid, to another object. The amount of liquid may be adjustable. The liquid handling system may specifically comprise one or more pipetting units. The pipetting unit may comprise at least one chamber being configured for holding or receiving at least one liquid. The pipetting unit may be configured for creating a partial vacuum above the chamber and for selectively releasing the partial vacuum to draw up and dispense the liquid. Further, additionally or alternatively, the liquid handling system may comprise at least one acoustic droplet ejection unit. The acoustic droplet ejection unit may be configured for using a pulse of ultrasound to move volumes of fluids without any physical contact. However, also other embodiments of the liquid handling system may be feasible.

The term "holder" as used herein is a broad term and is to be given its ordinary and customary meaning to a person of ordinary skill in the art and is not to be limited to a special or customized meaning. The term specifically may refer, without limitation, to an arbitrary device which may be configured for holding, specifically reversibly or releasable holding, an element. Specifically, the fiber sheet material may be impacted between at least two surfaces of the holder. The holder may specifically be or may comprise at least one pliers. However, also other embodiments may be feasible. The holder may specifically be configured for holding the at least one fiber sheet material having the at least one tip, wherein the holder is further configured for contacting the tip of the fiber sheet material to the sample comprising the analyte-microparticle-complex whereby at least the analyte-microparticle-complex is sucked into the tip of the fiber sheet material.

The term "sample changer" as used herein is a broad term and is to be given its ordinary and customary meaning to a person of ordinary skill in the art and is not to be limited to a special or customized meaning. The term specifically may refer, without limitation, to an arbitrary device which may be configured for transporting or moving an element into a desired position. Specifically, the sample changer may be configured for moving a plurality of elements successively into the desired position. The sample changer may specifically be an automated sample changer. The electrical contact may specifically be configured for applying the electrical voltage to the fiber sheet material whereby ions of the analyte are generated and are expelled from the tip of the fiber sheet material. The term "extracting solvent supply" as used herein is a broad term and is to be given its ordinary and customary meaning to a person of ordinary skill in the art and is not to be limited to a special or customized meaning. The term specifically may refer, without limitation, to an arbitrary device which is configured for applying an extracting solvent to an arbitrary object. Exemplarily, the extracting solvent supply may be or may comprise at least one pipetting unit. Further, the extracting solvent supply may be or may comprise a nozzle configured for spraying the extracting solvent onto the fiber sheet material. Further, extracting solvent supply may be or may comprise at least one capillary which is configured for providing a continuous flow of the extracting solvent onto the fiber sheet material. Also other embodiments may be feasible.

The system comprises a kit as disclosed.

The term "kit" as used herein is a broad term and is to be given its ordinary and customary meaning to a person of ordinary skill in the art and is not to be limited to a special or customized meaning. The term specifically may refer, without limitation, to a set of several items such as chemicals which are utilized for conducting a desired method, specifically for a sample preparation method and/or a sample analysis method. Specifically, the items may be provided in a housing, e.g. a packaging.

The kit comprises: (i) microparticles; (ii) at least one fiber sheet material having at least one tip; and (iii) at least one extracting solvent. With regard to the microparticles, the fiber sheet material and the extracting solvent reference to the description above is made.

The kit may optionally comprise further elements. The further elements may be selected from the group consisting of: a washing solvent, an internal standard; an auxiliary reagent, specifically a derivatization reagent.

Specifically, the microparticles, the fiber sheet material and the extracting solvent may be comprised in a housing. Moreover, also the further elements may be comprised in the housing.

The methods and devices according to the present invention provide a large number of advantages over known methods and devices.

Specifically, a microparticle workflow may be processed up to a point after a last washing step which removes unwanted matrix from analyte loaded microparticles. The analyte loaded microparticles are further sucked into the fiber sheet material, specifically into a membrane tissue. The fiber sheet material may also be referred to as paper fiber filter, cellulose fiber filter or as glass fiber filter.

Surprisingly, the analyte loaded microparticles, specifically the analyte-microparticle-complexes, only move during the sucking process a few millimeters into the fiber sheet material. After sucking the analyte loaded microparticles into the fiber sheet material the fiber sheet material may be dried and, optionally, even stored. Further, surprisingly, the microparticles may stay on the fiber sheet material and may not get into the port of the analytical device.

Moreover, an extraction of the microparticles occurs without using a pipettor. Thus, an additional step may be saved. Further, less solvent needs to be applied. Thus, costs for conducting the method are reduced.

For analysis, a supported microparticle spray based setup may be chosen in which a high voltage is applied to the dried fiber sheet material having the analyte-microparticle-complex in front of the tip. An extracting or electrospray ionization solvent may be applied onto an end of the fiber sheet material, which moves by capillary forces towards the tip of the fiber sheet material where the microparticles are located. Due to the extracting or electrospray ionization solvent an extraction of the microparticles on the fiber sheet material in a very tiny volume may occur instantly. The microparticles may stay on the tip due to their size and may not leave the fiber sheet material. By having the high voltage on the tip, the analytes get ionized and are therefore applicable for the mass spectrometer. The fiber sheet material may therefore act as a disposable for the prepared sample by using the microparticle workflow.

Any microparticles, e.g. octadecylsilane column material, solid phase extraction cartridge material, magnetic micro particles and DNA/RNA preparing particles may be used up to approx. 0.5 µm particle size. Principally, smaller sized particles can migrate through the paper sheet material and do not stay at the front of the tip.

Summarizing and without excluding further possible embodiments, the following embodiments may be envisaged:
The method for detecting at least one analyte in a sample comprises the following steps:
a) providing at least one sample having at least one analyte;
b) incubating the sample with microparticles having at least one surface whereby the analyte is adsorbed on the surface of the microparticles and an analyte-microparticle-complex is formed;
c) providing at least one fiber sheet material having at least one tip and contacting the tip of the fiber sheet material to the sample comprising the analyte-microparticle-complex whereby at least the analyte-microparticle-complex is sucked into the tip of the fiber sheet material;
d) contacting the tip of the fiber sheet material to a port of an analytical device;
e) adding an extracting solvent to the fiber sheet material and applying an electrical voltage to the fiber sheet material whereby ions of the analyte are generated and are expelled from the tip of the fiber sheet material; and
f) detecting the at least one analyte with the analytical device.

The analytical device may be selected from the group consisting of: a mass spectrometer; an ion mobility device; a combination of a mass spectrometer and an ion mobility device.

In step b) the sample may be incubated with the microparticles with an incubation time of 1 s to 60 min, preferably of 1 min to 30 min, most preferably of 3 min to 12 min.

The method may further comprise the following steps:
b1) separating of the analyte-microparticle-complex from further components of the sample; and
b2) removing the further components of the sample from the analyte-microparticle complex.

Step b1) may comprise a centrifugation of the sample.

The microparticles may be magnetic microparticles and step b1) may comprise a magnetic based separation.

The the method may further comprise the following step:
b3) washing the analyte-microparticle-complex.

The method may further comprise the following step:
c1) drying the fiber sheet material such that liquid of the sample evaporates.

The method may further comprise a storing of the fiber sheet material before step d) is conducted.

In step e) the extracting solvent may be applied onto the fiber sheet material.

In step e) the fiber sheet material may be in contact with a capillary, wherein a continuous flow of the extracting solvent is provided to the fiber sheet material via the capillary.

The continuous flow of the extracting solvent may have a flow rate of 1 µL/min to 50 µL/min, preferably of 10 µL/min to 30 µL/min, most preferably of 20 µL/min.

In step e), an electrical voltage of 1 kV to 10 kV, preferably of 2 kV to 5 kV, most preferably of 3 kV, may be applied.

The fiber sheet material may be provided as a strip.

The fiber sheet material may be selected from the group consisting of: cellulose, specifically a cellulose chromatography paper, specifically a cellulose acetate membrane; a glass filter membrane.

The fiber sheet material may have a grammage of 10 g/m² to 3000 g/m², preferably of 20 g/m² to 1000 g/m², most preferably of 80 g/m² to 700 g/m².

The tip of the fiber sheet material may have a triangular shape.

The tip may have a tip angle of 5° to 90°, preferably of 20° to 80°.

The microparticles may have a mean diameter of 100 nm to 100 µm, preferably of 200 nm to 50 µm.

The microparticles may be selected from the group consisting of: magnetic microparticles, specifically magnetic microparticles having a magnetic core and a modified surface; silica microparticles, specifically silica microparticles having a silica core and a modified surface; melamine resin microparticles, specifically melamine resin microparticles having a melamine resin core and a modified surface; poly(styrene) based microparticles, specifically poly(styrene) based microparticles having a poly(styrene) core and a modified surface; poly(methyl methacrylate) microparticles, specifically poly(methyl methacrylate) microparticles having a poly(methyl methacrylate) core and a modified surface.

The modified surface of the magnetic microparticles may be a modified poly(styrene) surface and The magnetic microparticles may have a mean diameter of 5 µm to 50 µm, preferably of 10 µm to 30 µm, most preferably of 20 µm.

The modified surface of the magnetic microparticles may be a silica surface and the magnetic microparticles may have a mean diameter of 100 nm to 1000 nm, preferably of 200 nm to 500 nm, most preferably of 300 nm.

The modified surface of the silica microparticles may be a cyanopropyl silane functionalized surface and the silica microparticles may have a mean diameter of 5 µm to 100 µm, preferably of 20 µm to 80 µm, most preferably of 40 µm.

The sample may be a biological sample, wherein the biological sample may be selected from the group consisting of: blood, serum, plasma, saliva, ocular lens fluid, cerebral spinal fluid, sweat, urine, milk, ascites fluid, mucous, synovial fluid, peritoneal fluid, amniotic fluid, tissue, cells.

The analyte may be selected from the group consisting of: a steroid, specifically a ketosteroid, specifically a secosteroid; a therapeutically active substance, specifically an antibiotic, specifically a local anesthetic, specifically Lidocaine; a peptide, specifically Cyclosporine A; a protein; a metabolite, specifically a nucleotide, specifically Adenosine 5'-monophosphate; a nucleic acid; an amino acid; a hormone; a fatty acid; a lipid; a carbohydrate.

A concentration of the analyte in the sample may be 0.01 pg/ml to 1 mg/ml, preferably 0.05 pg/ml to 50 µg/ml, most preferably 0.1 pg/ml to 10 µg/ml.

The analyte may have a molar mass from 10 to 10000 Da.

The tip of the fiber sheet material may be contacted to the port of the analytical device by one of the following procedures selected from the group consisting of: the tip of the fiber sheet material may be directly contacted to the port of the analytical device, specifically the tip of the fiber sheet material and the port of the analytical device may touch each other; the tip of the fiber sheet material may be indirectly contacted to the port of the analytical device, specifically the tip of the fiber sheet material and the port of the analytical device may be arranged in a distance to each other, preferably in a distance of 0.1 cm to 5 cm, preferably in a distance of 0.2 cm to 3 cm, most preferably in a distance of 0.5 cm to 2 cm.

A sample preparation system, specifically a continuous sample preparation system comprises:
- at least one vessel, wherein the vessel is configured for providing at least one sample having at least one analyte;
- at least one liquid handling system, wherein the liquid handling system is configured for adding microparticles to the vessel whereby the analyte is adsorbed on a surface of the microparticles and an analyte-microparticle-complex is formed;
- at least one holder, wherein the holder is configured for holding at least one fiber sheet material having at least one tip, wherein the holder is further configured for contacting the tip of the fiber sheet material to the sample comprising the analyte-microparticle-complex;
- at least one analytical device, specifically at least one mass spectrometer, wherein the analytical device is configured for detecting the at least one analyte, wherein the analytical device comprises at least one port;
- at least one sample changer, wherein the sample changer is configured for transferring the holder holding the fiber sheet material to the port of the analytical device, such that the tip of the fiber sheet material is in contact with the port;
- at least one electrical contact, wherein the electrical contact is configured for applying an electrical voltage to the fiber sheet material;
- at least one extracting solvent supply, wherein the extracting solvent supply is configured for wetting the fiber sheet material with at least one extracting solvent; and
a kit comprising (i) microparticles; (ii) at least one fiber sheet material having at least one tip; (iii) at least one extracting solvent.

The microparticles, the fiber sheet material and the extracting solvent may be comprised in a housing.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figures 1A to 1H: show an exemplary method for detecting at least one analyte in a sample according to the present invention;
- Figures 2A to 2E: show parts of an exemplary method for detecting at least one analyte in a sample according to the present invention;
- Figures 3A to 3C: show an exemplary embodiment of a sample preparation system according to the present invention in different views;
- Figures 4A to 4F: show full scan mass spectra of Lidocaine on Bead A (Figure 4A), of the second washing solution (Figure 4B), of the first washing solution (Figure 4C), of the supernatant after precipitation with Bead A (Figure 4D), of Lidocaine with a concentration of 10 µg/mL (Figure 4E) and of a blank experiment (Figure 4F);
- Figure 5: shows a logarithmic application of the numbers of counts resulting from the product ion scan;
- Figures 6A to 6C: show relevant parts of full scan mass spectra of Lidocaine extracted and ionized from the surface of a Lidocaine-microparticle-complex using different microparticle materials;
- Figures 7A to 7C: show relevant parts of a full scan mass spectrum of 4-Hydroxy Alprazolam extracted and ionized from a surface of a 4-Hydroxy Alprazolam-microparticle-complex using different microparticle materials (Figures 7B and 7C) in comparison to 4-Hydroxy Alprazolam in neat (Figure 7A);
- Figures 8A to 8B: show relevant parts of a full scan mass spectrum of Cyclosporine A extracted and ionized from a surface of a Cyclosporine A-microparticle-complex (Figure 8B) in comparison to Cyclosporine A in neat (Figure 8A);
- Figures 9A to 9C: show relevant parts of full scan mass spectra of Adenosine 5'-mono-phosphate extracted and ionized from a surface of an Adenosine 5'-mono-phosphate-microparticle-complex using different microparticle materials (Figures 9A and 9B) and the respective product ion spectrum of Adenosine 5' monophosphate (Figure 9C); and
- Figures 10A to 10B: show relevant parts of the full scan mass spectrum of Lidocaine spiked in horse serum (Figure 10A) and the respective product ion spectrum (Figure 10B).

### Detailed description of the embodiments

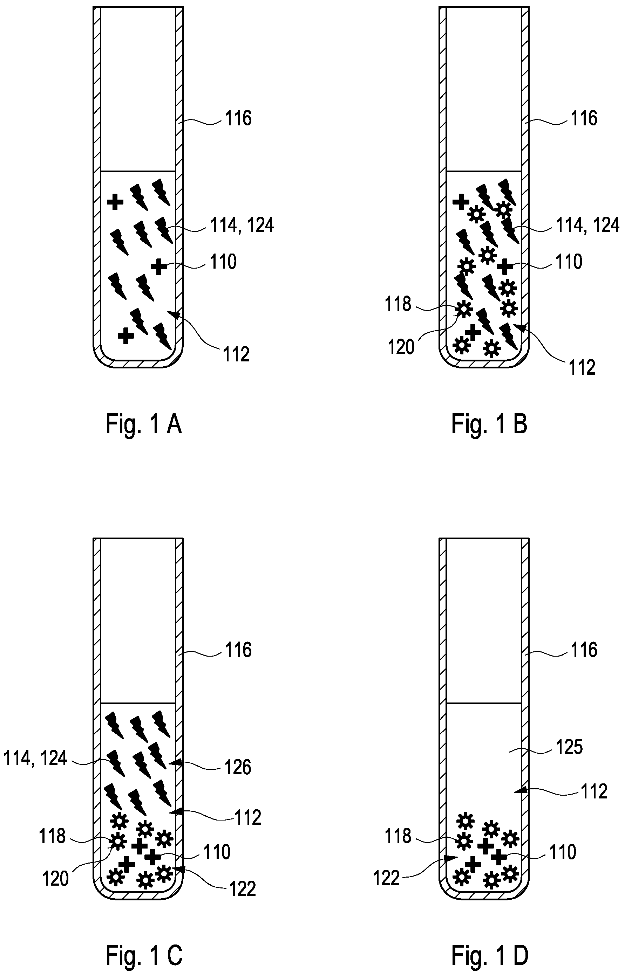

Figures 1A to 1H show an exemplary method for detecting at least one analyte 110 in a sample 112.

In step a), as illustrated in Figure 1A, the at least one sample 112 having the at least one analyte 110 is provided. The sample 112 may specifically be a biological sample which may comprise a matrix 114. Specifically, the sample 112 may be provided in a vessel 116.

Thereafter, microparticles 118 may be added to the sample 112. In step b), as illustrated in Figure 1B, the sample 112 is incubated with microparticles 118 having at least one surface 120 whereby the analyte 110 is adsorbed on the surface 120 of the microparticles 118 and an analyte-microparticle-complex 122 is formed. The analyte-microparticle-complex 122 is depicted in Figure 1C.

The step b) may further comprise, as illustrated in Figure 1C, a separating of the analyte-microparticle-complex 122 from further components 124 of the sample 112, specifically of the matrix 114, such as by centrifugation or magnetic based separation. Further, as illustrated in Figure 1D, the step b) may comprise a removing the further components 124 of the sample 112 from the analyte-microparticle complex 122.

Further, the step b) may comprise, as illustrated in Figure 1D, a washing of the analyte-microparticle complex 122. Thus, a washing solvent 125 may be added to the vessel 116.

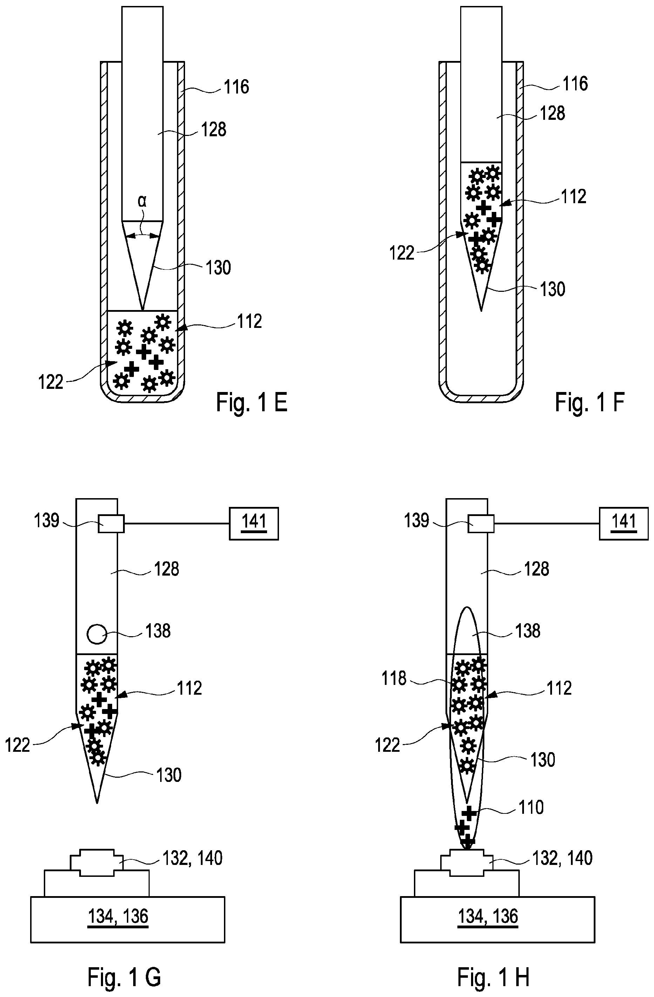

In step c), as illustrated in Figure 1E, at least one fiber sheet material 128 having at least one tip 130 is provided. The tip 130 may have a tip angle α of 10° to 30°, specifically of 20°.

Further, in step c), as illustrated in Figure 1F, the tip 130 of the fiber sheet material 128 is contacted to the sample 112 comprising the analyte-microparticle-complex 122 whereby at least the analyte-microparticle-complex 122 is sucked into the tip 130 of the fiber sheet material 128. For this purpose, the fiber sheet material 128 may be inserted at least partially into the vessel 116.

Thereafter, optionally, the fiber sheet material 128 may be dried such that liquid of the sample 112 evaporates (not shown). Further, optionally, the fiber sheet material 128 may be stored before step d), which is described below, is conducted.

In steps d) and e), as illustrated in Figures 1G and 1H, the tip 130 of the fiber sheet material 128 is connected to a port 132 of an analytical device 134. The analytical device 134 may specifically be a mass spectrometer 136. Thus, the port 132 may also be referred to as mass spectrometer entrance 140. Further, an extracting solvent 138 is added to the fiber sheet material 128 and an electrical voltage is applied to the fiber sheet material 128 whereby ions of the analyte 110 are generated and are expelled from the tip 130 of the fiber sheet material 128. Specifically, the electrical voltage is applied to the fiber sheet material 128 via at least one clip 139. The clip 139 may be connected to a voltage source supply 141.The extracting solvent 138 may specifically wet the whole fiber sheet material 128. Further, the extracting solvent 138 may dose the extraction of the microparticles 118, may also get ionized and may also be accelerated towards the port 132. As illustrated in Figure 1H, the microparticles 118 may stay on the tip 130 of the fiber sheet material 128.

Thereafter, the at least one analyte 110 is detected with the analytical device 134.

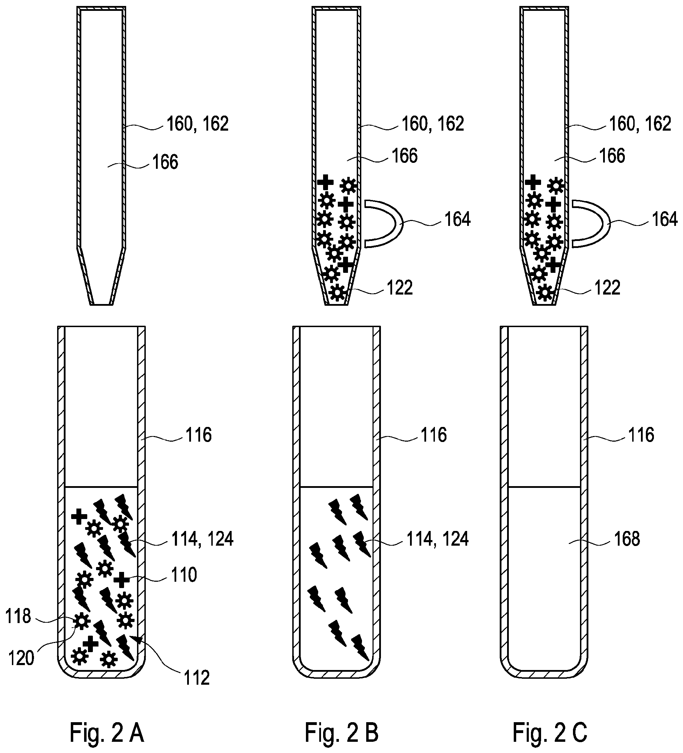

Figures 2A to 2E show parts of an exemplary method for detecting at least one analyte 110 in a sample 112 according to the present invention.

As illustrated in Figure 2B, the sample 112 is incubated with microparticles 118 having at least one surface 120. Thus, the analyte 110 is adsorbed on the surface 120 of the microparticles 118 and an analyte-microparticle-complex 122 is formed (depicted in Figure 2B). The step as illustrated in Figure 2A corresponds to step b) of the method for detecting at least one analyte 110 according to the present invention. Further, as illustrated in Figure 2B, at least one liquid handling device 160 such as at least one pipette 162 may be provided.

In a next step, as illustrated in Figure 2B, at least the analyte-microparticle-complex 122 may be sucked up by the liquid handling device 160. Thereafter, as illustrated in Figure 2B, a magnetic separator 164 may be brought in close proximity to the liquid handling device 160. The magnetic separator 164 may be configured for holding the analyte-microparticle-complex 122 within an interior space 166 of the liquid handling device 160. Thus, residual components of the sample 112 such as the matrix 114 may be separated from the analyte-microparticle-complex 122 and may be removed. As illustrated in Figure 2B, the residual components of the sample 112 such as the matrix 114 may be collected in the vessel 116.

Thereafter, optionally, washing steps may be conducted. For this purpose, the analyte-microparticle-complex 122 may be washed with at least one washing solvent 168 followed by a further sucking up of at least the analyte-microparticle-complex 122 by the liquid handling device 160 and by holding the analyte-microparticle-complex 122 within the interior space 166 of the liquid handling device 160 by the magnetic separator 164. As illustrated in Figure 2C, the washing solvent 168 may be collected in the vessel 116.

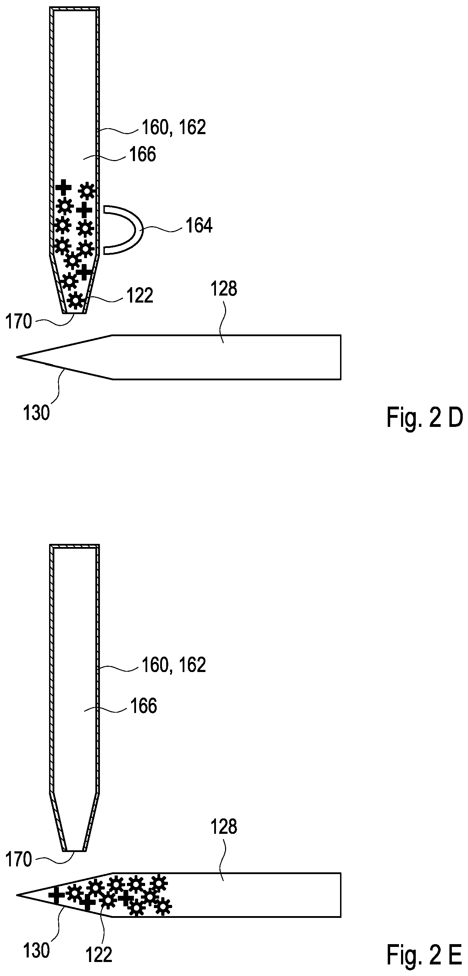

In a next step, as illustrated in Figure 2D, the at least one fiber sheet material 128 having the at least one tip 130 is provided. Further, as illustrated in Figure 2E, the tip 130 of the fiber sheet material 128 is contacted to the analyte-microparticle-complex 122 whereby at least the analyte-microparticle-complex 122 is sucked into the tip 130 of the fiber sheet material 128. For this purpose, the tip 130 of the fiber sheet material 128 may be brought close to a tip 170 of the liquid handling device 160. This step corresponds to step c) of the method for detecting at least one analyte 110 according to the present invention.

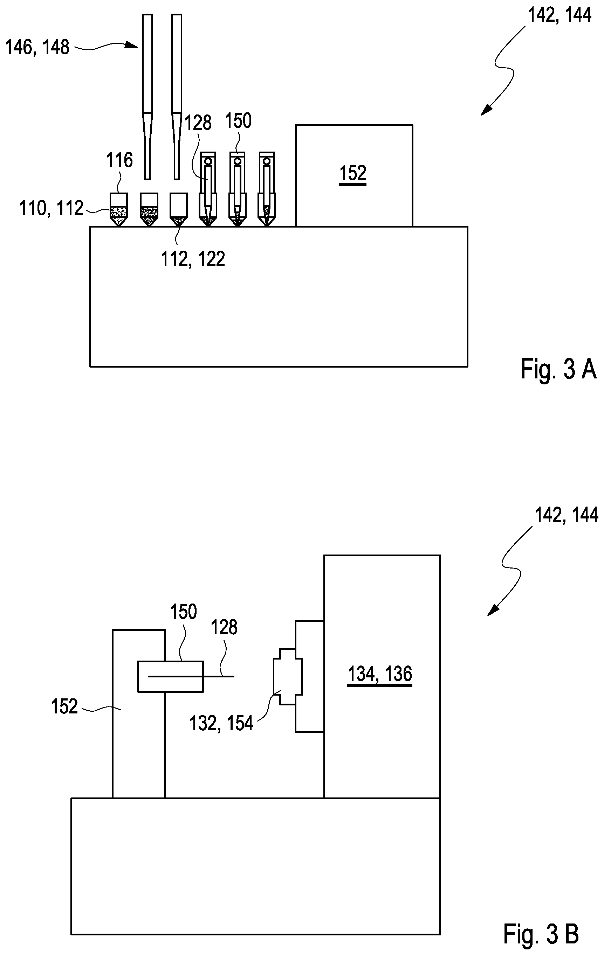

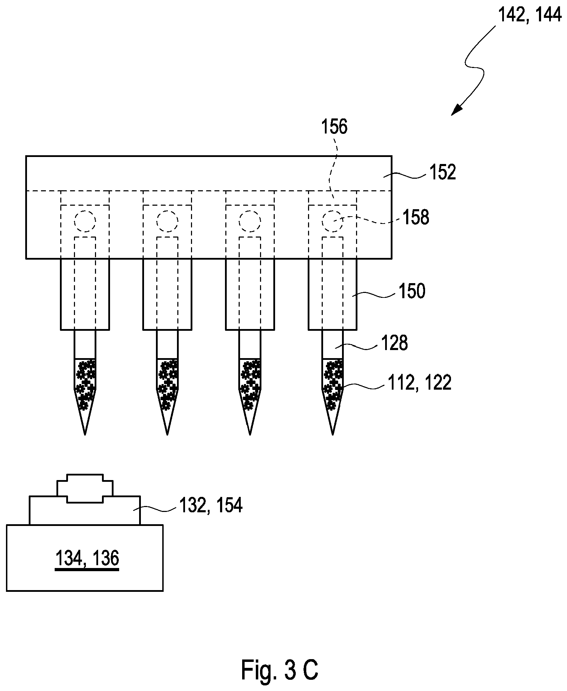

Figures 3A to 3C show an exemplary embodiment of a sample preparation system 142 according to the present invention. The sample preparation system 142 is schematically depicted in different views. Specifically, Figure 3A shows a front view, Figure 3B shows a side view and Figure 3C shows a top view of a part of the sample preparation system 142. The sample preparation system 142 may specifically be an automated sample preparation system 144.

As illustrated in Figure 3A, the sample preparation system 142 comprises the at least one vessel 116. Specifically, the sample preparation system may comprise a plurality of the vessels 116. The vessel 116 is configured for providing the at least one sample 112 having the at least one analyte 110. Further, the sample preparation system 142 comprises at least one liquid handling system 146 such as at least one pipetting unit 148. The pipetting unit 148 is configured for adding the microparticles 118 to the vessel 116 whereby the analyte 110 is adsorbed on the surface 120 of the microparticles 118 and the analyte-microparticle-complex 122 is formed. Further, the liquid handling system 146 may be configured for conducting further optional steps such as a removing of the further components 124 of the sample 112 from the analyte-microparticle-complex 122 and/or a washing of the analyte-microparticle-complex 122.

Further, the sample preparation system 142 comprises at least one holder 150. The holder 150 is configured for holding the at least one fiber sheet material 128 having the at least one tip 130. The holder 150 is further configured for contacting the tip 130 of the fiber sheet material 128 to the sample 112 comprising the analyte-microparticle-complex 122. The sample 112 comprising the analyte-microparticle-complex 122 may be sucked onto fiber sheet material 128 by capillary forces.

Further, as depicted in Figure 3A, the sample preparation system 142 comprises at least one sample changer 152. The sample changer 152 is configured for transferring the holder 150 holding the fiber sheet material 128 to the port 132 of the analytical device 134, such that the tip 130 of the fiber sheet material 128 is in contact with the port 132. The analytical device 134 of the sample preparation system 142 is depicted in further detail in Figures 3B and 3C.

As illustrated in Figure 3B, the sample changer 152 may be configured for positioning the holder 150 holding the fiber sheet material 128 to the port 132 of the analytical device 134. The port 132, which may also be referred to as ion block assembly 154, may be configured for allowing the ions to enter the mass spectrometer 136. The mass spectrometer 136 may comprise a quadrupole with subsequent ion trapping, isobaric separation via ion mobility, fragmentation in a collision cell and may be followed by quadrupole or time-of-flight (ToF) mass analysis. Other techniques of ion manipulations, like magnetic sector, and different combinations of the corresponding units may also be possible.

In Figure 3C, an electrical contact 156 is depicted which may be configured for ensuring a connection with a high voltage. Further, an extracting solvent supply 158 while a solvent supply may ensure a wetting of the fiber sheet material 128 with the extracting solvent.

### Examples

The following examples serve to illustrate the invention. They must not be interpreted as limiting with regard to the scope of protection.

All measurements were performed on a Xevo G2-XS Q-ToF mass spectrometer (Waters Corp.) where a modified 2D desorption electrospray ionization (DESI) source (Prosolia Inc.) was installed. The modification of the DESI source involved the replacement of the DESI spray head with an alligator clip that was connected to the high voltage source supply. A continuous extracting solvent flow was introduced by a syringe pump (Harvard Apparatus) with a capillary touching a rear side of the paper sheet material, specifically of the fiber sheet material.

Different paper sheet materials were tested such as cellulose chromatography paper in different grammage grades (87 g/m², 185 g/m², and 700 g/m², Whatman/Cytiva), as well as a glass filter membrane (64 g/m², Whatman/Cytiva), a Nylon membrane (Whatman/Cytiva), a PTFE membrane (Whatman/Cytiva) and a cellulose acetate membrane (What-man/Cytiva). For further measurements, the cellulose based membrane substrate with a grammage of 185 g/m² was used with a tip angle between 60°-90°, optimally of 80°.

As microparticles variations in the surface material as well as in the core material and the particle size were chosen. Therefore, two synthesized magnetic microparticles, specifically magnetic beads, namely 'Bead A' and 'LIAT', as well as commercial available particles from solid phase extraction (SPE) cartridges were used. Bead A comprises a magnetic inner core and a modified poly(styrene) surface with a size distribution around 20 µm. LIAT beads are smaller particles - commonly used for RNA/DNA purification - comprising a magnetic inner core and a SiO₂ surface with size distributions around 300 nm. Furthermore, commercial available SPE-Cyano (Bakerbond, Avantor Inc.) particles which are commonly known for capturing a broad range of polar to apolar molecules were used.

These SPE-Cyano particles comprise a silica core modified with a cyanopropyl silane functionalization and a size distribution around 40 µm.

The applied analytes were each dissolved in a low organic solvent mixture, optimally water and acetonitrile (H₂O/ACN=90/10). As analytes therapeutic or metabolic relevant molecules comprising different functional groups were chosen, namely Lidocaine (Lid), Cyclosporine A (CsA), 4-Hydroxy Alprazolam (4OHAlp) and Adenosine 5'-monophosphate (AMP). The main observed analyte adducts in positive ionization mode were as follows: m/z 235.2 [Lid+H]⁺, m/z 298.1 [4OHAlp-CN]⁺, m/z 325.1 [4OHAlp+H]⁺, m/z 1202.8 [CsA+H]⁺, m/z 1224.8 [CsA+Na]⁺, m/z 1240.8 [CsA+K]⁺. The observed analyte in the negative ionization mode was m/z 346.0 [AMP-H]⁻.

The mass spectra as described below in more detail show a relative abundance *ra* in % in dependence of the mass-to-charge ratio *m*/*z.*

### Example 1

As a first example a microparticle workflow was conducted.

A fast and dependable adsorption of the desired analytes on the microparticle surface during incubation and specifically during an enrichment and purification process was required. Furthermore, it was required that the analyte-particle-complex was sucked into the tip of the fiber sheet material, providing a fast extraction/desorption of analytes during the measurement.

In order to demonstrate the successful formation of the analyte-microparticle-complex, a model workflow applying Bead A (40 µL aqueous suspension) to the therapeutically substance Lidocaine (10 µg/mL, 150 µL, H₂O/ACN=90/10) was examined in detail. The single working steps were as follows: Incubation of the microparticles with the analyte solution (10 min), magnetic separation of the microparticles, removal of the supernatant and washing two times with water (150 µL) with magnetic separation and removal of the washing solutions inbetween. The residual analyte-microparticle-complex suspension was loaded onto the tip of the paper sheet material by sucking and drying. The microparticle loaded paper sheet material was introduced to the port, specifically to an ionization source, prewetted with 20 µL extracting solvent (ACN + 0.1% FA) and the measurement was started by applying a voltage of 3 kV at the rear side, specifically at a back side, of the paper sheet material. A continuous extraction solvent flow of 20 µL/min granted a constant wetting of the paper sheet material during the measurement.

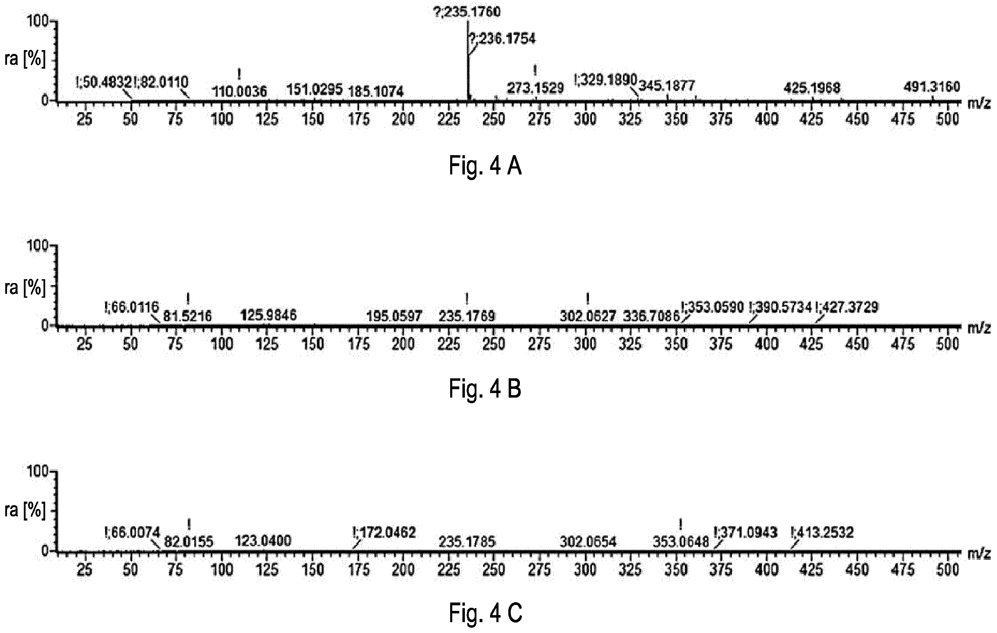

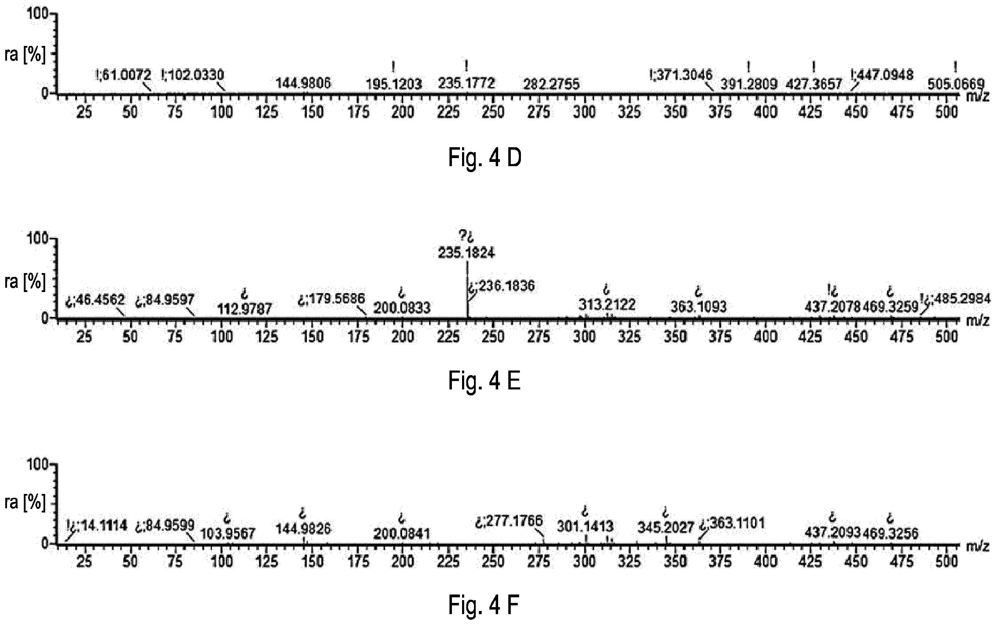

Figures 4A to 4F show different full scan mass spectra. Specifically, Figure 4A shows a full scan mass spectrum of Lidocaine on Bead A, Figure 4B shows a full scan mass spectrum of the second washing solution, Figure 4C shows a full scan mass spectrum of the first washing solution, Figure 4D shows a full scan mass spectrum of the supernatant after precipitation with Bead A, Figure 4E shows a full scan mass spectrum of Lidocaine with a concentration of 10 µg/mL and Figure 4F shows a full scan mass spectrum of a blank experiment.

The blank experiment as illustrated in Figure 4F includes a measurement just with the extracting solvent on a fiber sheet material. For the experiments according to Figures 4B to 4E a sample was respectively spotted in a volume of 5 µL directly on the tip of the fiber sheet material, dried and measured with the same settings as with the analyte-bead-complex before. The intensity axes of the mass spectra were normalized to the highest signal overall. The protonated analyte signals [Lid+H]⁺ at m/z 235.2 were additionally selected and fragmented (collision energy CE = 20 eV). Resulting product ion scans obtained almost exclusively the ionic fragment [CH₂N(C₂H₅)₂]⁺ at m/z 86.1.

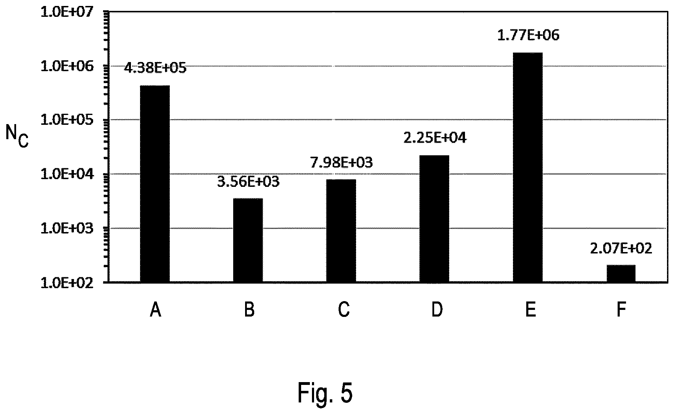

Figure 5 shows a logarithmic application of the numbers of counts *N_{c}* resulting from the product ion scan from m/z 235.2 to m/z 86.1 (collision energy CE = 20 eV). A corresponds to Lidocaine on Bead A, B corresponds to the second washing solution, C corresponds to the first washing solution, D corresponds to the supernatant after precipitation with Bead A. E corresponds to Lidocaine with a concentration of 10 µg/mL and *F* corresponds to the blank experiment. Thus, in Figure 5, the number of counts *N_{c}* of Lidocaine loaded on Bead A can be compared to the corresponding bead workflow steps in a logarithmic application. It can clearly be observed that the concentration of Lidocaine remaining in the supernatant after the bead depletion step decreases in a magnitude of about 100. This proves the favorable formation of the analyte-microparticle-complex. During the two washing steps, the concentration of Lidocaine in the supernatant solution reduces further to a level of a few 1000 counts. Sucking and drying of the analyte-microparticle-complex on the tip of the paper sheet material and following the same measurement conditions resulted in the extraction and ionization of the analyte from the microparticle surface.

### Example 2

As a second example different microparticle materials were utilized.

To demonstrate a broad applicability of present invention, different microparticles were tested. This variation comprised the size of the microparticles as well as the solid support core material and the surface material. The micorparticle workflows were applied as previously described, except for using SPE-Cyano, where the precipitation of microparticles was not conducted magnetically, but with the use of a benchtop centrifuge.

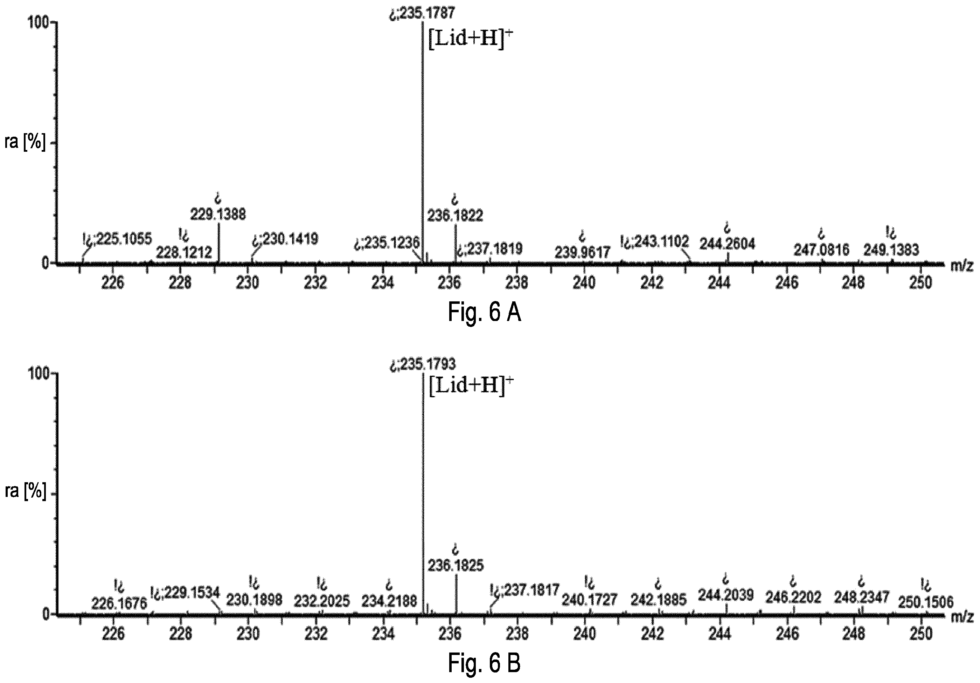

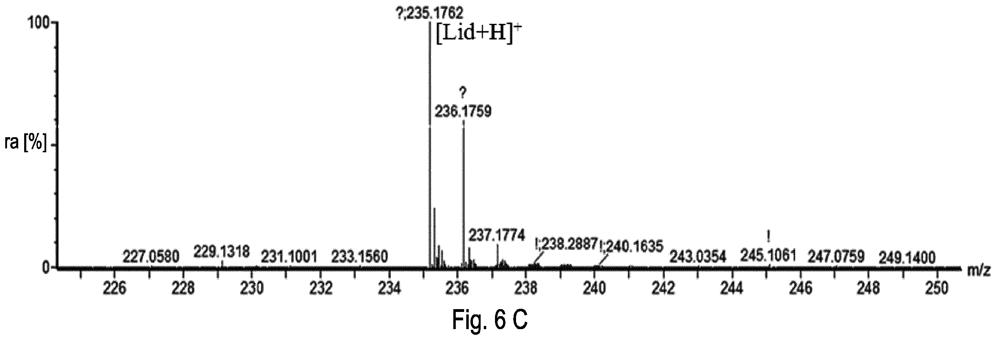

Figures 6A to 6C show relevant parts of full scan mass spectra (m/z 224 to 251) of Lidocaine extracted and ionized from the surface of a Lidocaine-microparticle-complex using different microparticle materials. Figure 6A shows the relevant part of a full scan mass spectrum wherein LIAT beads were utilized. Figure 6B shows the relevant part of a full scan mass spectrum wherein SPE-Cyano beads were utilized. Figure 6C shows the relevant part of a full scan mass spectrum wherein beads A were utilized. Specifically, Figures 6A to 6C respectively show the relevant parts of the full scan mass spectrum of Lidocaine [Lid+H]⁺ at m/z 235.2 resulting from the supported microparticle spray after the enrichment of the analytes with the corresponding bead workflows. Bead A showed thereby the best performance, followed by SPE-Cyano. Even the use of LIAT beads, that are normally known to enrich RNA/DNA or negatively charged/ionizing molecules, showed hereby some formation of the Lidocaine-LIAT complex and desorption/ionization, but as expected with lower intensities compared to Bead A. It must also be added, that the high number of ionized analytes - especially while using Bead A - resulted in a saturation of the detector, which can be observed especially with the higher than natural isotope signal at m/z 236.2. The actual number of counts are therefore expected to be even higher than displayed and underpin further the good enrichment and ionization of the herein described method.

### Example 3

As a third example different analytes were applied.

To demonstrate the broad applicability of the herein described invention, a further set of analytes were tested, representing a variety of chemical functionalities (e.g. peptide, hydroxyl, halide, aryl, heterocycle or nucleotide) as well as positive and negative ionization. Therefore, 4-Hydroxy Alprazolam (4OHAlp), Cyclosporine A (CsA) and Adenosine 5'-monophosphate (AMP) were selected. All workflows were applied as previously described, except for using SPE-Cyano, where the precipitation of microparticles was not magnetically conducted, but with the use of a benchtop centrifuge.

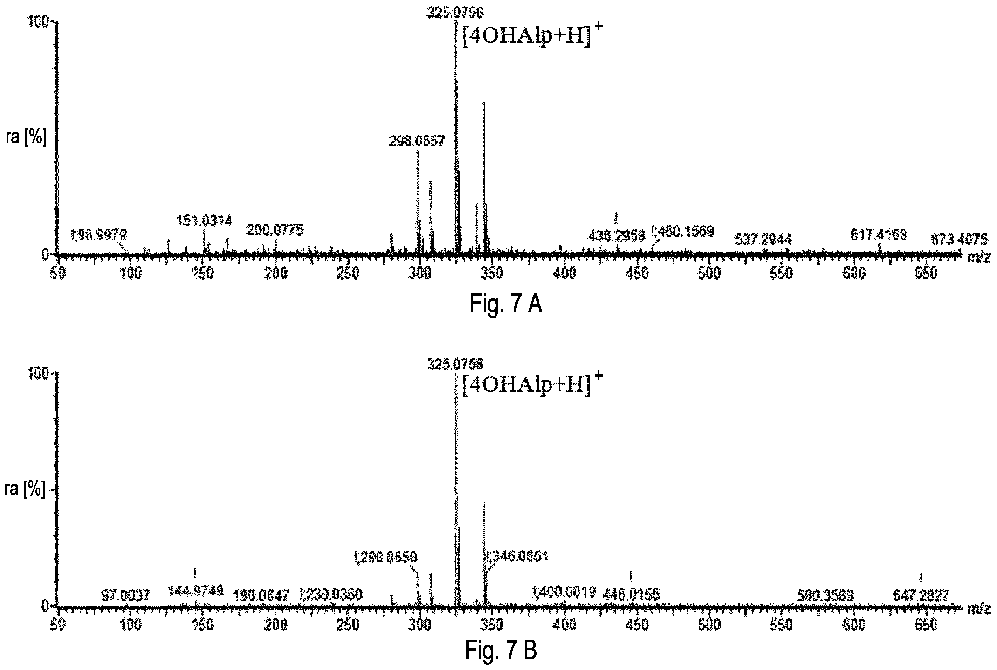

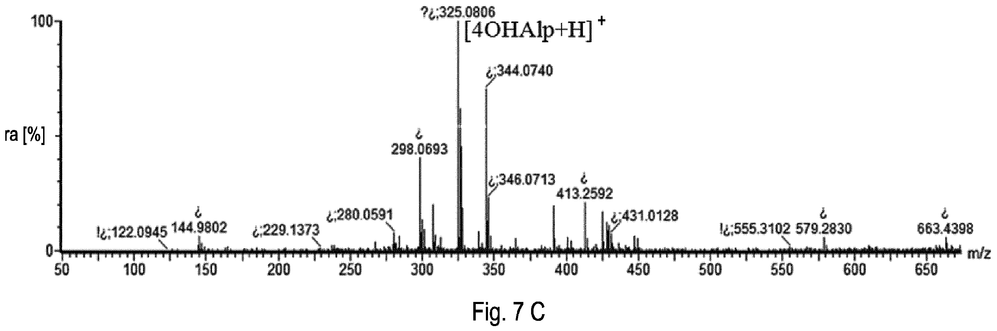

Figures 7A to 7C show relevant parts of a full scan mass spectrum (m/z 50 to 675) of 4-Hydroxy Alprazolam extracted and ionized from a surface of a 4-Hydroxy Alprazolam-microparticle-complex using different microparticle materials (Figures 7B and 7C) in comparison to 4-Hydroxy Alprazolam in neat (Figure 7A). Figure 7A shows a relevant part of a full scan mass spectrum of 4-Hydroxy Alprazolam wherein a direct sampling of 5 µL of a 10 µg/mL 4OHAlp on the fiber sheet material was conducted. Figure 7B shows a relevant part of a full scan mass spectrum of 4-Hydroxy Alprazolam extracted and ionized from the surface of a 4OHAlp-microparticle-complex using Bead A. Figure 7C shows a relevant part of a full scan mass spectrum of 4-Hydroxy Alprazolam extracted and ionized from the surface of a 4OHAlp-microparticle-complex using SPE-Cyano. Specifically, Fig-ures 7A to 7C show the relevant parts of the full scan mass spectra of 4-Hydroxy Alprazolam [4OHAlp+H]⁺ at m/z 325.1. Interestingly, the SPE-Cyano material showed the best performance in the enrichment and desorption of 4OHAlp.

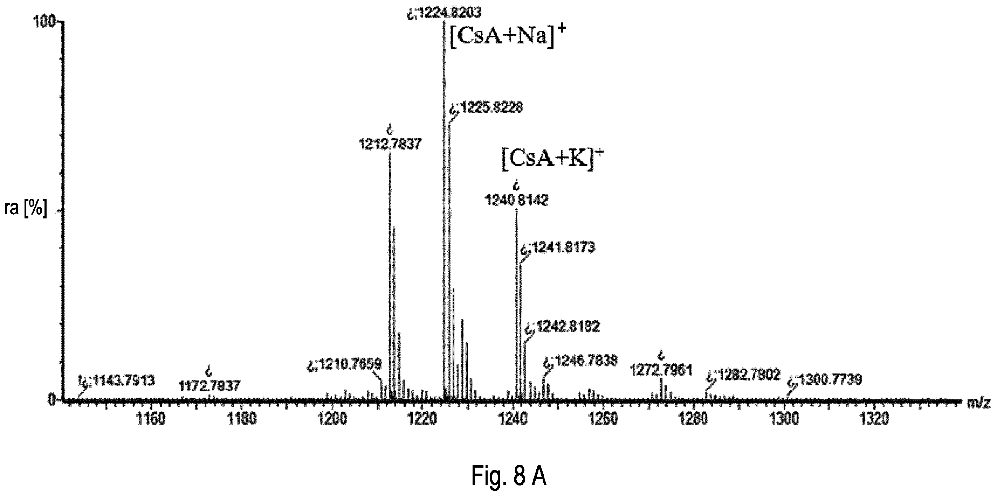

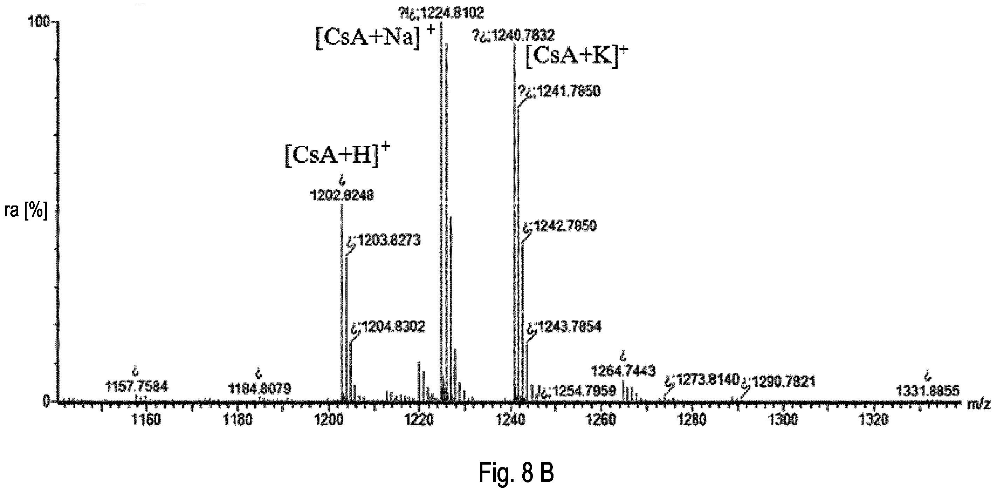

Furthermore, Cyclosporine A was absorbed on Bead A. The bead workflow was applied as previously described. Figures 8A and 8B show relevant parts of a full scan mass spectrum (m/z 1140 to 1340) of Cyclosporine A (CsA). Figures 8A and 8B show relevant parts of a full scan mass spectrum of Cyclosporine A extracted and ionized from a surface of a Cyclosporine A-microparticle-complex (Figure 8B) in comparison to Cyclosporine A in neat (Figure 7A). Figure 8A shows a relevant part of a full scan mass spectrum of CsA, wherein a direct sampling of 5 µL of CsA having a concentration of 10 µL/mL on the paper sheet material was conducted.

Figure 8B shows a relevant part of a full scan mass spectrum of CsA extracted and ionized from the surface from a CsA-microparticle-complex using Bead A. Bead A thereby showed good results in the enrichment of CsA, supporting also the subsequent extraction and ionization of the analyte during the measurement.

A compatibility with analytes that prefer a negative ionization would also be beneficial in terms of the broadness of application. For this the nucleotide Adenosine 5'-monophosphate (AMP) was selected which represents an important metabolic compound and is commonly known for also showing a negative ionization. The enrichment of AMP (10 µg/mL, 150 µL, H₂O/ACN= 90/10) on LIAT and Bead A was gained by the bead workflow as described before. The residual analyte-microparticle-complex suspension was each loaded onto the tip of the paper sheet material by sucking and drying. The paper sheet material was introduced to the supported bead spray ionization source, prewetted with 20 µL extracting solvent (ACN + 0.1% Tributylamine) and the measurement was started by applying a negative voltage of 2 kV at the rear side, specifically the back, of the paper sheet material. A continuous extracting solvent flow of 20 µL/min granted a constant wetting of the paper sheet material during the measurement.

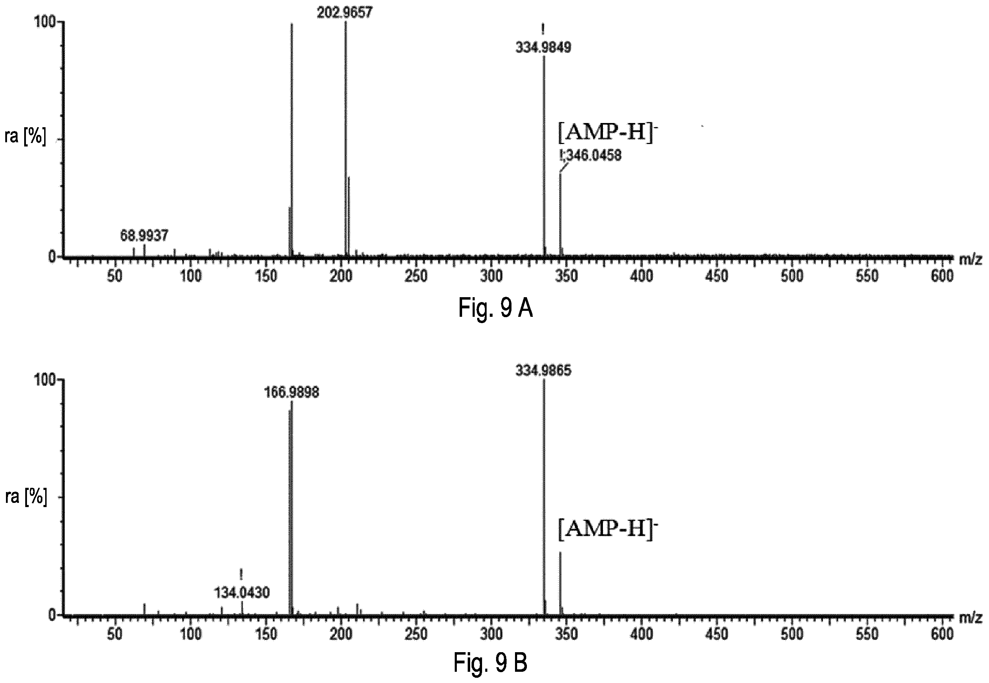

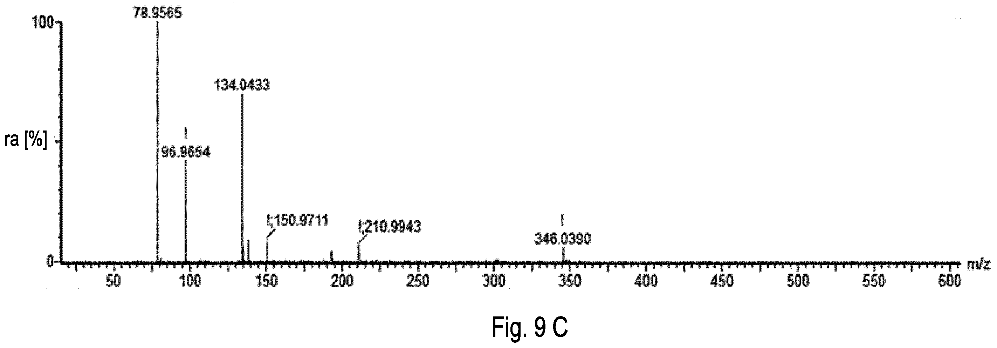

Figures 9A to 9C show relevant parts of full scan mass spectra (m/z 20 to 610) of Adeno-sine 5' monophosphate extracted and ionized from a surface of an Adenosine 5' mono-phosphate-microparticle-complex using different microparticle materials (Figures 9A and 9B) and the respective product ion spectrum of Adeno-sine 5' monophosphate (Figure 9C). For Figure 9A, Adenosine 5' mono-phosphate was extracted and ionized from the surface of an AMP-microparticle-complex using Bead A in the negative ion mode. For Figure 9B Adenosine 5'-monophosphate was extracted and ionized from the surface of an AMP-microparticle-complex using LIAT beads in the negative ion mode. The negative product ion represented [AMP-H]⁻ at m/z 346.0 and Figure 9C shows the product ion scan of this corresponding anion on LIAT beads after the application of a collision energy of 25 eV, resulting in the commonly known fragments of AMP. Other dominant signals in both full scan mass spectra at m/z 167.0, m/z 203.0 and m/z 335.0 belong presumably to the surfactant 4-phosphonobutyric acid, which is used in the manufacturing of cellulose-based chromatography paper and represents an impurity in all paper based fiber sheet materials. The performance of AMP on LIAT was quite better, compared to Bead A, which was also expected, because LIAT beads are specifically designed to capture negatively charged or polarized analytes of interests.

### Example 4

As a fourth example a performance in the presence of a realistic matrix was evaluated.

An important use of this invention is the enrichment and ionization of analytes, which are present in a challenging matrix, like whole blood, serum or urine. To combine the herein described method with a realistic matrix, an initial analyte solution comprising 150 µL horse serum that was spiked with Lidocaine (1 µg/mL), was utilized. The solution was pretreated with 50 µL of a 30% MeOH, vortexed and incubated for 10 min. The bead workflow using Bead A was performed as described before. The extracting solvent comprised ACN + 0.1% FA and the measurement was performed in positive ionization mode by applying a voltage of 3.5 kV at the rear side, specifically the back, of the paper sheet material.

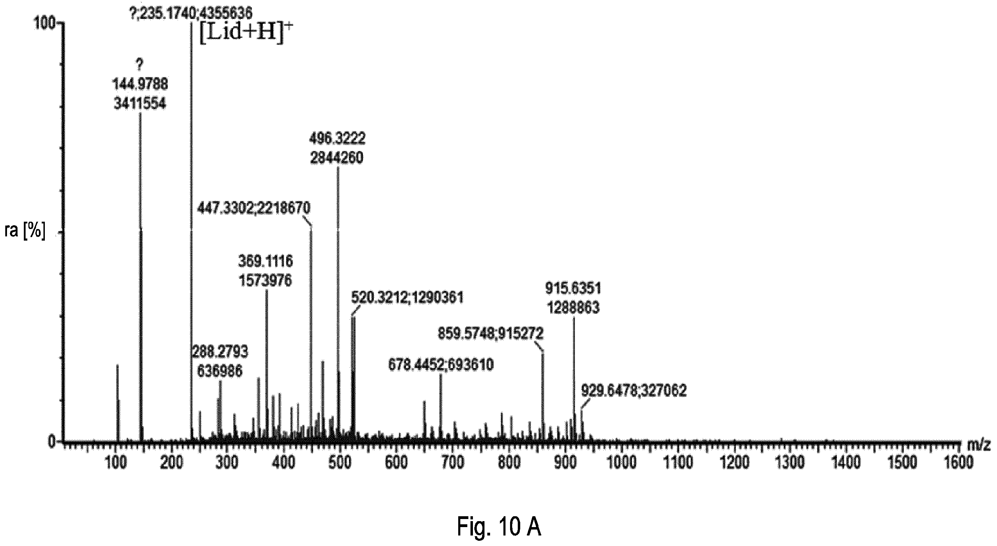

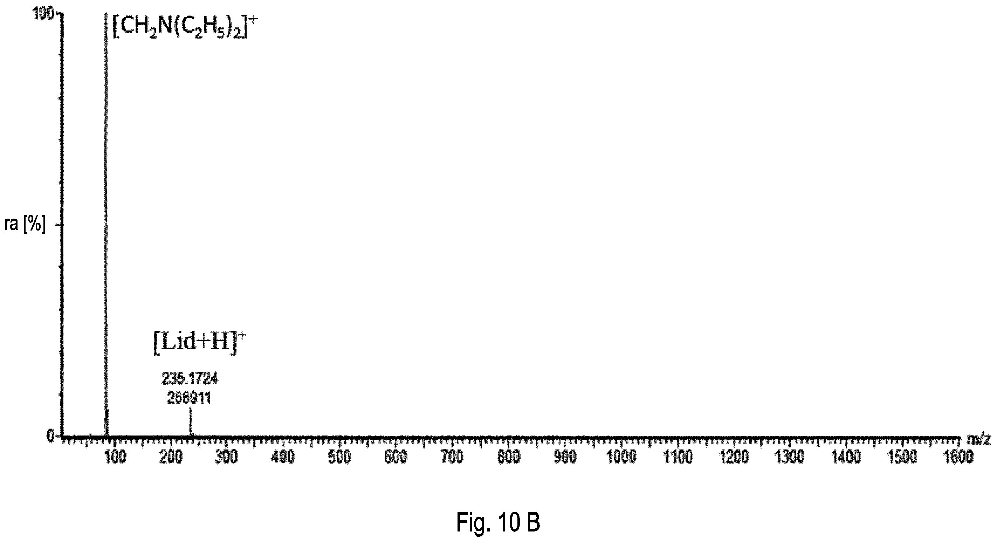

Figures 10A and 10B show relevant parts of the full scan mass spectrum (m/z 20 to 1600) of Lidocaine (1 µg/mL). For Figure 10A Lidocaine (1 µg/mL) was spiked in horse serum after extraction and ionization from a surface of a Lidocaine-microparticle-complex using Bead-A. In Figure 10B a product ion scan of this measurement is displayed. The product ion scan of Lidocaine from horse serum enriched on Bead A (m/z 325.2 to 86.1, CE = 20 eV) clearly approved the presence of Lidocaine in its protonated form [Lid+H]⁺. These results prove the successful enrichment, purification and ionization of a specific analyte out of a challenging matrix, such as horse serum with the possibility of its quantification by the use of a supported bead spray ionization measurement.

### List of reference numbers

- 110: analyte
- 112: sample
- 114: matrix
- 116: vessel
- 118: microparticle
- 120: surface
- 122: analyte-microparticle-complex
- 124: further component
- 125: washing solvent
- 126: supernatant
- 128: fiber sheet material
- 130: tip
- 132: port
- 134: analytical device
- 136: mass spectrometer
- 138: extracting solvent
- 139: clip
- 140: mass spectrometer entrance
- 141: voltage source supply
- 142: sample preparation system
- 144: automated sample preparation system
- 146: liquid handling system
- 148: pipetting unit
- 150: holder
- 152: sample changer
- 154: ion block assembly
- 156: electrical contact
- 158: extracting solvent supply
- 160: liquid handling device
- 162: pipette
- 164: magnetic separator
- 166: interior space
- 168: washing solvent
- 170: tip

## Claims

1. A method for detecting at least one analyte (110) in a sample (112), wherein the method comprises the following steps:
a) providing at least one sample (112) having at least one analyte (110);
b) incubating the sample (112) with microparticles (118) having at least one surface (120) whereby the analyte (110) is adsorbed on the surface (120) of the microparticles (118) and an analyte-microparticle-complex (122) is formed;
c) providing at least one fiber sheet material (128) having at least one tip (130) and contacting the tip (130) of the fiber sheet material (128) to the sample (112) comprising the analyte-microparticle-complex (122) whereby at least the analyte-microparticle-complex (122) is sucked into the tip (130) of the fiber sheet material (128);
d) contacting the tip (130) of the fiber sheet material (128) to a port of an analytical device, wherein contacting in the context of step d) comprises at least one of: being brought into contact; direct contacting; indirect contacting; arranged in distance to each other;
e) adding an extracting solvent (138) to the fiber sheet material (128) and applying an electrical voltage to the fiber sheet material (128) whereby ions of the analyte (110) are generated and are expelled from the tip (130) of the fiber sheet material (128); and
f) detecting the at least one analyte (110) with the analytical device (134).

2. The method according to the preceding claim, wherein the analytical device (134) is selected from the group consisting of: a mass spectrometer (136); an ion mobility device; a combination of a mass spectrometer and an ion mobility device.

3. The method according to any one of the preceding claims, wherein the method further comprises the following steps:
b1) separating of the analyte-microparticle-complex (122) from further components (124) of the sample (112); and
b2) removing the further components (124) of the sample (112) from the analyte-microparticle complex (122).

4. The method according to any one of the preceding claims, wherein the method further comprises a storing of the fiber sheet material (128) before step d) is conducted.

5. The method according to any one of the preceding claims, wherein in step e) the fiber sheet material (128) is in contact with a capillary, wherein a continuous flow of the extracting solvent (138) is provided to the fiber sheet material (128) via the capillary.

6. The method according to the preceding claim, wherein the continuous flow of the extracting solvent (138) has a flow rate of 1 µL/min to 50 µL/min.

7. The method according to any one of the preceding claims, wherein the fiber sheet material (128) is selected from the group consisting of: cellulose; a glass filter membrane.

8. The method according to any one of the preceding claims, wherein the fiber sheet material (128) has a grammage of 10 g/m² to 3000 g/m², preferably of 20 g/m² to 1000 g/m², most preferably of 80 g/m² to 700 g/m².

9. The method according to any one of the preceding claims, wherein the microparticles (118) have a mean diameter of 100 nm to 100 µm.

10. The method according to any one of the preceding claims, wherein the microparticles (118) are selected from the group consisting of: magnetic microparticles; silica microparticles; melamine resin microparticles; poly(styrene) based microparticles; poly(methyl methacrylate) microparticles.

11. The method according to any one of the preceding claims, wherein the sample (112) is a biological sample, wherein the biological sample is selected from the group consisting of: blood, serum, plasma, saliva, ocular lens fluid, cerebral spinal fluid, sweat, urine, milk, ascites fluid, mucous, synovial fluid, peritoneal fluid, amniotic fluid, tissue, cells.

12. The method according to any one of the preceding claims, wherein the analyte (110) is selected from the group consisting of: a steroid; a therapeutically active substance; a peptide; a metabolite.

13. The method according to any one of the preceding claims, wherein a concentration of the analyte (110) in the sample (112) is 0.01 pg/ml to 1 mg/ml.

14. A sample preparation system (142), wherein the sample preparation system (142) comprises:
• at least one vessel (116), wherein the vessel (116) is configured for providing at least one sample (112) having at least one analyte (110);
• at least one liquid handling system (146), wherein the liquid handling system (146) is configured for adding microparticles (118) to the vessel (116) whereby the analyte (110) is adsorbed on a surface (120) of the microparticles (118) and an analyte-microparticle-complex (122) is formed;
• at least one holder (150), wherein the holder (150) is configured for holding at least one fiber sheet material (128) having at least one tip (130), wherein the holder (150) is further configured for contacting the tip (130) of the fiber sheet material (128) to the sample (112) comprising the analyte-microparticle-complex (122);
• at least one analytical device (134), wherein the analytical device (134) is configured for detecting the at least one analyte (110), wherein the analytical device (134) comprises at least one port (132);
• at least one sample changer (152), wherein the sample changer (152) is configured for transferring the holder (150) holding the fiber sheet material (128) to the port (132) of the analytical device (134), such that the tip (130) of the fiber sheet material (128) is in contact with the port (132);
• at least one electrical contact (156), wherein the electrical contact (156) is configured for applying an electrical voltage to the fiber sheet material (128);
• at least one extracting solvent supply (158), wherein the extracting solvent supply (158) is configured for wetting the fiber sheet material (128) with at least one extracting solvent (138); and
• a kit comprising (i) microparticles (118); (ii) at least one fiber sheet material (128) having at least one tip (130); (iii) at least one extracting solvent (138),
wherein the sample preparation system is configured for conducting the method according to any one of the claims 1 to 13.

## Patentansprüche

1. Verfahren zum Nachweis mindestens eines Analyten (110) in einer Probe (112), wobei das Verfahren die folgenden Schritte umfasst:
a) Bereitstellen mindestens einer Probe (112) mit mindestens einem Analyten (110);
b) Inkubieren der Probe (112) mit Mikropartikeln (118) mit mindestens einer Oberfläche (120), wodurch der Analyt (110) an der Oberfläche (120) der Mikropartikel (118) adsorbiert wird und ein Analyt-Mikropartikel-Komplex (122) gebildet wird;
c) Bereitstellen mindestens eines Faserblattmaterials (128) mit mindestens einer Spitze (130) und Inkontaktbringen der Spitze (130) des Faserblattmaterials (128) mit der Probe (112), die den Analyt-Mikropartikel-Komplex (122) umfasst, wodurch mindestens der Analyt-Mikropartikel-Komplex (122) in die Spitze (130) des Faserblattmaterials (128) gesaugt wird;
d) Inkontaktbringen der Spitze (130) des Faserblattmaterials (128) mit einem Anschluss einer Analysevorrichtung, wobei das Inkontaktbringen im Rahmen von Schritt d) mindestens eines aus Folgendem umfasst: in Kontakt gebracht werden; direktes Inkontaktbringen; indirektes Inkontaktbringen; im Abstand zueinander angeordnet;
e) Hinzufügen eines Extraktionslösungsmittels (138) zu dem Faserblattmaterial (128) und Anlegen einer elektrischen Spannung an das Faserblattmaterial (128), wodurch Ionen des Analyten (110) erzeugt und aus der Spitze (130) des Faserblattmaterials (128) ausgestoßen werden; und
f) Nachweisen des mindestens einen Analyten (110) mit der Analysevorrichtung (134).

2. Verfahren nach dem vorhergehenden Anspruch, wobei die Analysevorrichtung (134) ausgewählt ist aus der Gruppe bestehend aus: einem Massenspektrometer (136); einer Ionenmobilitätsvorrichtung; einer Kombination aus einem Massenspektrometer und einer Ionenmobilitätsvorrichtung.

3. Verfahren nach einem der vorhergehenden Ansprüche, wobei das Verfahren ferner die folgenden Schritte umfasst:
b1) Trennen des Analyt-Mikropartikel-Komplexes (122) von weiteren Komponenten (124) der Probe (112); und
b2) Entfernen der weiteren Komponenten (124) der Probe (112) aus dem Analyt-Mikropartikel-Komplex (122).

4. Verfahren nach einem der vorhergehenden Ansprüche, wobei das Verfahren ferner ein Lagern des Faserblattmaterials (128) umfasst, bevor Schritt d) durchgeführt wird.

5. Verfahren nach einem der vorhergehenden Ansprüche, wobei in Schritt e) das Faserblattmaterial (128) mit einer Kapillare in Kontakt steht, wobei dem Faserblattmaterial (128) ein kontinuierlicher Fluss des Extraktionslösungsmittels (138) über die Kapillare bereitgestellt wird.

6. Verfahren nach dem vorhergehenden Anspruch, wobei der kontinuierliche Fluss des Extraktionslösungsmittels (138) eine Flussrate von 1 µl/min bis 50 µl/min aufweist.

7. Verfahren nach einem der vorhergehenden Ansprüche, wobei das Faserblattmaterial (128) ausgewählt ist aus der Gruppe bestehend aus: Cellulose; einer Glasfiltermembran.

8. Verfahren nach einem der vorhergehenden Ansprüche, wobei das Faserblattmaterial (128) ein Flächengewicht von 10 g/m² bis 3000 g/m², vorzugsweise von 20 g/m² bis 1000 g/m², am meisten bevorzugt von 80 g/m² bis 700 g/m² aufweist.

9. Verfahren nach einem der vorhergehenden Ansprüche, wobei die Mikropartikel (118) einen mittleren Durchmesser von 100 nm bis 100 µm aufweisen.

10. Verfahren nach einem der vorhergehenden Ansprüche, wobei die Mikropartikel (118) ausgewählt sind aus der Gruppe bestehend aus: magnetischen Mikropartikeln; Siliciumdioxid-Mikropartikeln; Melaminharz-Mikropartikeln; Mikropartikeln auf Poly(styrol)-Basis; Poly(methylmethacrylat)-Mikropartikeln.

11. Verfahren nach einem der vorhergehenden Ansprüche, wobei die Probe (112) eine biologische Probe ist, wobei die biologische Probe ausgewählt ist aus der Gruppe bestehend aus: Blut, Serum, Plasma, Speichel, Augenlinsenflüssigkeit, Zerebrospinalflüssigkeit, Schweiß, Urin, Milch, Aszitesflüssigkeit, Schleim, Synovialflüssigkeit, Peritonealflüssigkeit, Fruchtwasser, Gewebe, Zellen.

12. Verfahren nach einem der vorhergehenden Ansprüche, wobei der Analyt (110) ausgewählt ist aus der Gruppe bestehend aus: einem Steroid; einer therapeutisch aktiven Substanz; einem Peptid; einem Metaboliten.

13. Verfahren nach einem der vorhergehenden Ansprüche, wobei eine Konzentration des Analyten (110) in der Probe (112) 0,01 pg/ml bis 1 mg/ml beträgt.

14. Probenvorbereitungssystem (142), wobei das Probenvorbereitungssystem (142) Folgendes umfasst:
• mindestens ein Gefäß (116), wobei das Gefäß (116) zum Bereitstellen mindestens einer Probe (112) mit mindestens einem Analyten (110) konfiguriert ist;
• mindestens ein Flüssigkeitshandhabungssystem (146), wobei das Flüssigkeitshandhabungssystem (146) zum Hinzufügen von Mikropartikeln (118) in das Gefäß (116) konfiguriert ist, wodurch der Analyt (110) an einer Oberfläche (120) der Mikropartikel (118) adsorbiert wird und ein Analyt-Mikropartikel-Komplex (122) gebildet wird;
• mindestens einen Halter (150), wobei der Halter (150) zum Halten mindestens eines Faserblattmaterials (128) mit mindestens einer Spitze (130) konfiguriert ist, wobei der Halter (150) ferner zum Inkontaktbringen der Spitze (130) des Faserblattmaterials (128) mit der Probe (112), die den Analyt-Mikropartikel-Komplex (122) umfasst, konfiguriert ist;
• mindestens eine Analysevorrichtung (134), wobei die Analysevorrichtung (134) zum Nachweisen des mindestens einen Analyten (110) konfiguriert ist, wobei die Analysevorrichtung (134) mindestens einen Anschluss (132) umfasst;
• mindestens einen Probenwechsler (152), wobei der Probenwechsler (152) zum Überführen des Halters (150), der das Faserblattmaterial (128) hält, zu dem Anschluss (132) der Analysevorrichtung (134) konfiguriert ist, sodass die Spitze (130) des Faserblattmaterials (128) mit dem Anschluss (132) in Kontakt steht;
• mindestens einen elektrischen Kontakt (156), wobei der elektrische Kontakt (156) zum Anlegen einer elektrischen Spannung an das Faserblattmaterial (128) konfiguriert ist;
• mindestens eine Extraktionslösungsmittelzufuhr (158), wobei die Extraktionslösungsmittelzufuhr (158) zum Benetzen des Faserblattmaterials (128) mit mindestens einem Extraktionslösungsmittel (138) konfiguriert ist; und
• einen Kit, umfassend (i) Mikropartikel (118); (ii) mindestens ein Faserblattmaterial (128) mit mindestens einer Spitze (130); (iii) mindestens ein Extraktionslösungsmittel (138),
wobei das Probenvorbereitungssystem zum Durchführen des Verfahrens nach einem der Ansprüche 1 bis 13 konfiguriert ist.

## Revendications

1. Procédé pour détecter au moins un analyte (110) dans un échantillon (112), dans lequel le procédé comprend les étapes suivantes :
a) fourniture d'au moins un échantillon (112) ayant au moins un analyte (110) ;
b) incubation de l'échantillon (112) avec des microparticules (118) ayant au moins une surface (120) moyennant quoi l'analyte (110) est adsorbé sur la surface (120) des microparticules (118) et un complexe analyte-microparticules (122) est formé ;
c) fourniture d'au moins un matériau en feuille de fibres (128) ayant au moins une pointe (130) et mise en contact de la pointe (130) du matériau en feuille de fibres (128) avec l'échantillon (112) comprenant le complexe analyte-microparticules (122) moyennant quoi au moins le complexe analyte-microparticules (122) est aspiré dans la pointe (130) du matériau en feuille de fibres (128) ;
d) mise en contact de la pointe (130) du matériau en feuille de fibres (128) avec un port d'un dispositif analytique, la mise en contact dans le contexte de l'étape d) comprenant au moins un parmi : l'entrée en contact ; la mise en contact direct ; la mise en contact indirect ; l'agencement à distance l'un de l'autre ;
e) ajout d'un solvant d'extraction (138) au matériau en feuille de fibres (128) et application d'une tension électrique au matériau en feuille de fibres (128) moyennant quoi des ions de l'analyte (110) sont générés et sont expulsés de la pointe (130) du matériau en feuille de fibres (128) ; et
f) détection de l'au moins un analyte (110) avec le dispositif analytique (134).

2. Procédé selon la revendication précédente, dans lequel le dispositif analytique (134) est choisi dans le groupe constitué par : un spectromètre de masse (136) ; un dispositif de mobilité ionique ; une association d'un spectromètre de masse et d'un dispositif de mobilité ionique.

3. Procédé selon l'une quelconque des revendications précédentes, dans lequel le procédé comprend en outre les étapes suivantes :
b1) séparation du complexe analyte-microparticules (122) d'autres composants (124) de l'échantillon (112) ; et
b2) élimination des autres composants (124) de l'échantillon (112) du complexe analyte-microparticules (122).

4. Procédé selon l'une quelconque des revendications précédentes, dans lequel le procédé comprend en outre un stockage du matériau en feuille de fibres (128) avant que l'étape d) ne soit réalisée.

5. Procédé selon l'une quelconque des revendications précédentes, dans lequel dans l'étape e) le matériau en feuille de fibres (128) est en contact avec un capillaire, dans lequel un flux continu du solvant d'extraction (138) est fourni au matériau en feuille de fibres (128) par l'intermédiaire du capillaire.

6. Procédé selon la revendication précédente, dans lequel le flux continu du solvant d'extraction (138) a un débit de 1 µL/min à 50 µL/min.

7. Procédé selon l'une quelconque des revendications précédentes, dans lequel le matériau en feuille de fibres (128) est choisi dans le groupe constitué par : la cellulose ; une membrane en filtre de verre.

8. Procédé selon l'une quelconque des revendications précédentes, dans lequel le matériau en feuille de fibres (128) a un grammage de 10 g/m² à 3000 g/m², de préférence de 20 g/m² à 1000 g/m², de manière préférée entre toutes de 80 g/m² à 700 g/m².

9. Procédé selon l'une quelconque des revendications précédentes, dans lequel les microparticules (118) ont un diamètre moyen de 100 nm à 100 µm.

10. Procédé selon l'une quelconque des revendications précédentes, dans lequel les microparticules (118) sont choisies dans le groupe constitué par : les microparticules magnétiques ; les microparticules de silice ; les microparticules de résine de mélamine ; les microparticules à base de poly(styrène) ; les microparticules de poly(méthacrylate de méthyle).

11. Procédé selon l'une quelconque des revendications précédentes, dans lequel l'échantillon (112) est un échantillon biologique, dans lequel l'échantillon biologique est choisi dans le groupe constitué par : le sang, le sérum, le plasma, la salive, le liquide du cristallin, le liquide céphalo-rachidien, la sueur, l'urine, le lait, le liquide d'ascite, du mucus, le liquide synovial, le liquide péritonéal, le liquide amniotique, un tissu, des cellules.

12. Procédé selon l'une quelconque des revendications précédentes, dans lequel l'analyte (110) est choisi dans le groupe constitué par : un stéroïde ; une substance thérapeutique active ; un peptide ; un métabolite.

13. Procédé selon l'une quelconque des revendications précédentes, dans lequel une concentration de l'analyte (110) dans l'échantillon (112) est de 0,01 pg/ml à 1 mg/ml.

14. Système de préparation d'échantillon (142), dans lequel le système de préparation d'échantillon (142) comprend :
• au moins un récipient (116), le récipient (116) étant configuré pour fournir au moins un échantillon (112) ayant au moins un analyte (110) ;
• au moins un système de manipulation de liquide (146), le système de manipulation de liquide (146) étant configuré pour ajouter des microparticules (118) au récipient (116) moyennant quoi l'analyte (110) est adsorbé sur une surface (120) des microparticules (118) et un complexe analyte-microparticules (122) est formé ;
• au moins un support (150), le support (150) étant configuré pour supporter au moins un matériau en feuille de fibres (128) ayant au moins une pointe (130), le support (150) étant en outre configuré pour mettre en contact la pointe (130) du matériau en feuille de fibres (128) avec l'échantillon (112) comprenant le complexe analyte-microparticules (122) ;
• au moins un dispositif analytique (134), le dispositif analytique (134) étant configuré pour détecter l'au moins un analyte (110), le dispositif analytique (134) comprenant au moins un port (132) ;
• au moins un passeur d'échantillons (152), le passeur d'échantillons (152) étant configuré pour transférer le support (150) supportant le matériau en feuille de fibres (128) jusqu'au port (132) du dispositif analytique (134), de telle sorte que la pointe (130) du matériau en feuille de fibres (128) est en contact avec le port (132) ;
• au moins un contact électrique (156), le contact électrique (156) étant configuré pour appliquer une tension électrique au matériau en feuille de fibres (128) ;
• au moins une alimentation en solvant d'extraction (158), l'alimentation en solvant d'extraction (158) étant configurée pour mouiller le matériau en feuille de fibres (128) avec au moins un solvant d'extraction (138) ; et
• un kit comprenant (i) des microparticules (118) ; (ii) au moins un matériau en feuille de fibres (128) ayant au moins une pointe (130) ; (iii) au moins un solvant d'extraction (138),
dans lequel le système de préparation d'échantillon est configuré pour mettre en œuvre le procédé selon l'une quelconque des revendications 1 à 13.
